# EUROPEAN PATENT APPLICATION

(11) **EP 1 356 815 A1**
(43) Date of publication of application: **29.10.2003**
(21) Application number: 02710361.3
(22) Date of filing: 28.01.2002
(51) Int. Cl.: A61K 31/4178, A61K 31/4184, A61K 31/4245, A61K 45/00, A61P 43/00, A61P 29/00, A61P 25/04, A61P 9/10, A61P 9/08, A61P 9/12, C07D 403/10

(54) **ANALGESIC AND ANTIINFLAMMATORY DRUGS**

(30) Priority: 29.01.2001 JP 2001020012
(71) Applicant: Takeda Chemical Industries, Ltd., Osaka-shi, Osaka 541-0045 (JP)
(72) Inventor: TERASHITA, Zen-ichi, Toyonaka-shi, Osaka 565-0085 (JP); NARUO, Ken-ichi, Sanda-shi, Hyogo 669-1535 (JP); MORIMOTO, Shigeru, Tondabayashi-shi, Osaka 584-0067 (JP)
(74) Representative: Best, Michael, Dr.
(86) International application number: JP0200596
(87) International publication number: WO02060439

(57) **Abstract**

Excellent analgesic and antiinflammatory agents comprising a compound having an angiotensin II antagonistic activity, its prodrugs thereof or salts of the same.

## Description

### Technical Field

The present invention relates to analgesic and antiinflammatory agent comprising, as an active ingredient, a compound having the angiotensin II antagonistic activity (All antagonistic activity), a prodrug thereof or a salt thereof.

### Background Art

As factors associated with inflammation and pain, there are known prostaglandins, leukotrienes, platelet activating factor, bradykinin, substance P, complement, cytokines, histamine, serotonine, active oxygen, nitrogen monoxide and lysosome enzyme. Compounds which regulate production or action of these factors are expected to exert the antiinflammatory action or the analgesic action. Main antiinflammatories which are currently used are roughly classified into steroidal antiinflammatories and non-steroidal antiinflammatories. Steroidal antiinflammatories are thought to be due to inhibition of production, liberation or activity of various mediators involved in inflammation. On the other hand, non-steroidal antiinflammatories are further classified into acidic and basic antiinflammatories, and both of which possess the analgesic, antiinflammatory and antipyretic activities. Acidic non-steroidal antiinflammatories are thought to inhibit cycloxygenase and exclude association of prostaglandins. Recently, cycloxygenase 2 selective inhibitors having little digestive tract disorder have been developed. The mechanism of action of basic antiinflammatories has not been made clear as compared with that of acidic non-steroidal antiinflammatories, and the extremely weak or little cycloxygenase inhibiting activity is perceived. Acetaminophen has the antipyretic and analgesic activity comparable to that of aspirin, but has the extremely weak antiinflammatory activity. Acetaminophen has the extremely weak cycloxygenase inhibiting activity.

As an analgesic drug, there is a group of substances, a representative which is an anesthetic analgesic drug which mainly acts on the central part and has the strong analgesic activity, in addition to a group of drugs which act mainly on the periphery part and have the mild analgesic activity, such as non-steroidal antiinflammatories. The anesthetic analgesic drug includes alkaloid contained in opium, morphine and codeine, and structurally associated synthetic drugs thereof, and is thought to exert the action via an opioid receptor.

On the other hand, compounds having the All antagonistic activity are known as a drug for treating circulatory diseases such as hypertension, cardiac diseases (hypercardia, cardiac failure and cardiac infarct), cerebral apoplexy and nephritis (JP-A 4-364171), and it is known that the persistent depressor activity by inhibition of the action of All having the strong vasopressor activity on an All receptor.

There is desired development of an analgesic agent and an antiinflammatory agent which are excellent in the direct analgesic activity and antiinflammatory activity, and have the sufficiently excellent nature such as no side effects as a drug.

### Disclosure of Invention

In view of the aforementioned circumstances, the present inventors intensively studied useful analgesic and antiinflammatory agents and, as a result, found that compounds having the angiotensin II antagonistic activity, in particular, compounds having the angiotensin II (AII) antagonistic activity represented by a particular structural formula exhibit the direct analgesic activity and antiinflammatory activity, further studied based on these findings, which resulted in completion of the present invention.

That is, the present invention relates to:
[1] an analgesic agent comprising 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof,
[2] an antiinflammatory agent comprising 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof,
[3] an analgesic agent comprising a compound having the angiotensin II antagonistic activity selected from Losartan, Candesartan, Olmesartan and Tasosartan, a prodrug thereof or a salt thereof,
[4] an antiinflammatory agent comprising a compound having the angiotensin II antagonistic activity selected from Candesartan, Telmisartan, Olmesartan and Tasosartan, a prodrug thereof or a salt thereof,
[5] an analgesic agent for pain associated with chronic inflammation or headache associated with hypertension, comprising a compound having the angiotensin II antagonistic activity, a prodrug thereof a salt thereof,
[6] an agent for improving a pain sense threshold comprising a compound having the angiotensin II antagonistic activity, a prodrug thereof or a salt thereof,
[7] an agent for preventing or treating (1) arteriosclerosis including atherosclerosis, (2) vascular hypertrophy, occlusion or organ disorder after intervention, (3) reocclusion, restenosis or endothelial functional disorder after bypass operation, (4) intermittent claudication, (5) occlusive peripheral circulation disorder, or (6) inflammatory disease or pain due to occlusive arteriosclerosis, comprising a compound having the angiotensin II antagonistic activity, a prodrug thereof or a salt thereof,
[8] the agents according to any one of the above-mentioned [5] to [7], wherein the compound having the angiotensin II antagonistic activity is a non-peptidic compound,
[9] the agents according to any one of the above-mentioned [5] to [7], wherein the compound having the angiotensin II antagonistic activity is a compound having an oxygen atom in the molecule,
[10] the agents according to any one of the above-mentioned [5] to [7], wherein the compound having the angiotensin II antagonistic activity is a compound having an ether linkage or a carbonyl group,
[11] the agents according to any one of the above-mentioned [5] to [7], wherein the compound having the angiotensin II antagonistic activity is a compound represented by the formula: 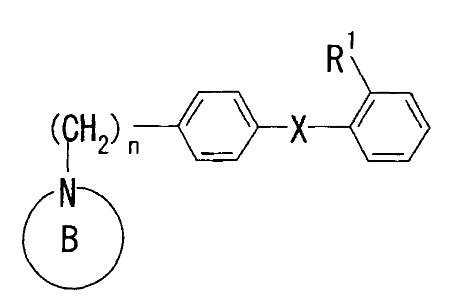 wherein a ring B denotes an optionally substituted nitrogen-containing heterocycle, R¹ denotes a group which can forms an anion or a group which can be converted into an anion, X denotes that the phenylene group and the phenyl group are bound directly or through a spacer having a chain length of 1 or 2 atoms, and n denotes an integer of 1 or 2,
[12] the agent according to the above-mentioned [11], wherein the ring B is an optionally substituted nitrogen-containing aromatic heterocycle,
[13] the agent according to the above-mentioned [11], wherein the ring B is an optionally substituted 5 or 6-membered nitrogen-containing heterocycle,
[14] the agent according to the above-mentioned [11], wherein the ring B is an optionally substituted imidazole ring,
[15] the agents according to any one of the above-mentioned [5] to [7], wherein the compound having the angiotensin II antagonistic activity is a compound represented by the formula (I): 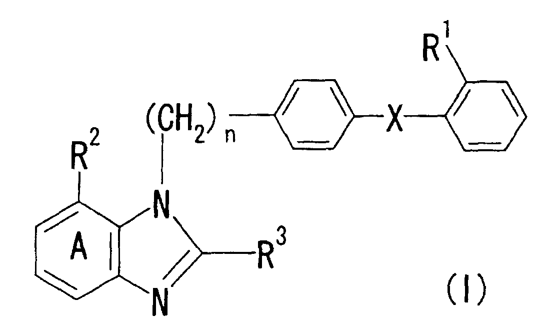 wherein R¹ denotes a group which can form an anion or a group which can be converted into an anion, X denotes that the phenylene group and the phenyl group are bound directly or through a spacer having a chain length of 1 or 2 atoms, n denotes 1 or 2, a ring A denotes a benzene ring optionally further having a substituent, R² denotes a group which can form an anion or a group which can be converted into an anion, and R³ denotes a hydrocarbon residue which may be bound via a hetero atom and which may have a substituent,
[16] the agent according to any one of the above-mentioned [5] to [7], wherein the compound having the angiotensin II antagonistic activity is Losartan, Eprosartan, Candesartan Cilexetil, Candesartan, Valsartan, Telmisartan, Irbesartan, Olmesartan or Tasosartan,
[17] the agents according to any one of the above-mentioned [5] to [7], wherein the compound having the angiotensin II antagonistic activity is 2-ethoxy-1-[[2'-(1H-tetrazol-5-yl)biphenyl-4-yl]methyl]benzimidazole-7-carboxylic acid,
[18] the agents according to any one of the above-mentioned [5] to [7], wherein the compound having the angiotensin II antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate,
[19] the agents according to any one of the above-mentioned [5] to [7], wherein the compound having the angiotensin II antagonistic activity is 2-ethoxy-1-[[2'-(5-oxo-2,5-dihydro-1,2,4-oxadiazol-3-yl)biphenyl-4-yl]methyl]-1H-benzimidazole-7-carboxylic acid,
[20] a method for preventing or treating pain, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
[21] a method for preventing or treating inflammatory disease, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
[22] a method for preventing or treating pain, which comprises administering an effective amount of a compound having the angiotensin II antagonistic activity selected from Losartan, Candesartan, Olmesartan and Tasosartan, a prodrug thereof or a salt thereof to a mammal,
[23] a method for preventing or treating inflammatory disease, which comprises administering an effective amount of a compound having the angiotensin II antagonistic activity selected from Candesartan, Telmisartan, Olmesartan and Tasosartan, a prodrug thereof or a salt thereof to a mammal,
[24] a method for preventing or treating pain associated with chronic inflammation or headache associated with hypertension, which comprises administering an effective amount of a compound having the angiotensin II antagonistic activity, a prodrug thereof or a salt thereof to a mammal,
[25] a method for improving a pain sense threshold, which comprises administering an effective amount of a compound having the angiotensin II antagonistic activity, a prodrug thereof or a salt thereof to a mammal,
[26] a method for preventing or treating (1) arteriosclerosis including atherosclerosis, (2) vascular hypertrophy, occlusion or organ disorder after intervention, (3) reocclusion, restenosis or endothelial functional disorder after bypass operation, (4) intermittent claudication, (5) occlusive peripheral circulation disorder, or (6) inflammatory disease or pain due to occlusive arteriosclerosis, which comprises administering an effective amount of a compound having the angiotensin II antagonistic activity, a prodrug thereof or a salt thereof to a mammal,
[27] use of 2-ethoxy-1-[[2'-(5-oxo-2,5-dihydro-1,2,4-oxadiazol-3-yl)biphenyl-4-yl] methyl]-1H-benzimidazole-7-carboxylic acid or a salt thereof for preparation of an analgesic agent,
[28] use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for preparation of an antiinflammatory agent,
[29] use of a compound having the angiotensin II antagonistic activity selected from Losartan, Candesartan, Olmesartan and Tasosartan, a prodrug thereof or a salt thereof for preparation of an analgesic agent,
[30] use of a compound having the angiotensin II antagonistic activity selected from Candesartan, Telmisartan, Olmesartan and Tasosartan, a prodrug thereof or a salt thereof for preparation of an antiinflammatory agent,
[31] use of a compound having the angiotensin II antagonistic activity, a prodrug thereof or a salt thereof for preparation of an analgesic agent for pain associated with chronic inflammation or headache associated with hypertension,
[32] use of a compound having the angiotensin II antagonistic activity, a prodrug thereof or a salt thereof for preparation of an agent for improving pain sense threshold, and
[33] use of a compound having the angiotensin II antagonistic activity, a prodrug thereof or a salt thereof for preparation of an agent for preventing or treating (1) arteriosclerosis including atherosclerosis, (2) vascular hypertrophy, occlusion or organ disorder after intervention, (3) reocclusion, restenosis or endothelial functional disorder after bypass operation, (4) intermittent claudication, (5) occlusive peripheral circulation disorder, or (6) inflammatory disease or pain due to occlusive arteriosclerosis.

A compound having the angiotensin II antagonistic activity, a prodrug thereof or a salt thereof in the present invention can be advantageously used as an analgesic agent or an antiinflammatory agent.

An angiotensin II antagonistic activity in the present invention refers to such the action that binding of angiotensin II to an angiotensin II receptor on a cell membrane is competitively or non-competitively inhibited to attenuate the strong vasoconstrictive activity and vascular smooth muscle growth activity induced by angiotensin II.

A compound having the angiotensin II antagonistic activity used in the preset invention may be peptidic or non-peptidic, and a non-peptidic compound having the angiotensin II antagonistic activity which is advantageous in long acting time is preferable. As a compound having the angiotensin II antagonistic activity, a compound having an oxygen atom in its molecule is preferable and, inter alia, a compound having an ether linkage or a carbonyl group (the carbonyl group may form a hydroxyl group by resonance) is preferable, a compound having an ether linkage or a ketone derivative is more preferable and, inter alia, an ether derivative is preferable.

A non-peptidic compound having the angiotensin II antagonistic activity is not particularly limited, and imidazole derivatives are disclosed in JP-A 56-71073, JP-A 56-71074, JP-A 57-98270, JP-A 58-157768, USP 4,355,040 and USP 4,340,598, improved imidazole derivatives are disclosed in EP-253310, EP-291969, EP-324377, EP-403158, WO-9100277, JP-A 63-23868 and JP-A 1-117876, pyrrole, pyrazole and triazole derivatives are disclosed in USP 5,183,899, EP-323841, EP-409332 and JP-A 1-287071, benzimidazole derivative are disclosed in USP4,880,804, EP-0392317, EP-0399732, EP-0400835, EP-425921, EP-459136 and JP-A 3-63264, azaindene derivatives are disclosed in EP-399731, pyrimidone derivatives are disclosed in EP-407342, quinazoline derivatives are disclosed in EP-411766, xanthine derivatives are disclosed in EP-430300, fused imidazole derivatives are disclosed in EP-434038, pyrimidinedione derivatives are disclosed in EP-442473, theinopyridone derivatives are disclosed in EP-443568, and heterocyclic compounds are disclosed in EP-445811, EP-483683, EP-518033, EP-520423, EP-588299 and EP-603712. Representative compounds of the above compounds are described in Journal of Medicinal chemistry, (Vol.39, No. 3, pp 625-656, 1996). As a non-peptidic compound having the angiotensin II antagonistic activity, in addition to the aforementioned compounds described in the known literatures, any compounds may be used as far as they are non-peptidic compounds having the angiotensin II antagonistic activity, inter alia, Losartan (DuP753), Losartan potassium, Eprosartan (SK&F108566), Candesartan cilexetil (TCV-116), Valsartan (CGP-48933), Telmisartan (BIBR277), Irbesartan (SR47436), Tasosartan (ANA-756), Olmesartan (CS-866) also called Olmesartan medoxomyl, Olmesartan (including RNH-6270) and active metabolites thereof (Candesartan etc.) are preferably used.

In addition, as a non-peptidic compound having the angiotensin II antagonistic activity, for example, a compound represented by the formula: 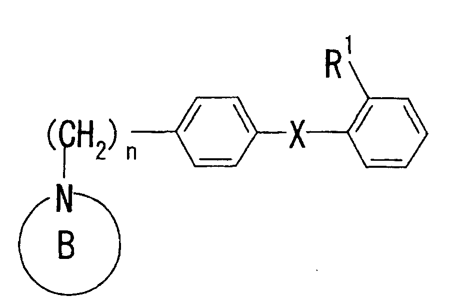 wherein a ring B denotes an optionally substituted nitrogen-containing heterocycle, R¹ denotes a group which can form an anion or a group which can be converted into the group, X denotes that a phenylene group and a phenyl group are bound directly or a spacer having no more than 2 of atom chains, and n denotes an integer of 1 or 2, or a salt thereof; or,
a benzimidazole derivative represented by the formula (I): 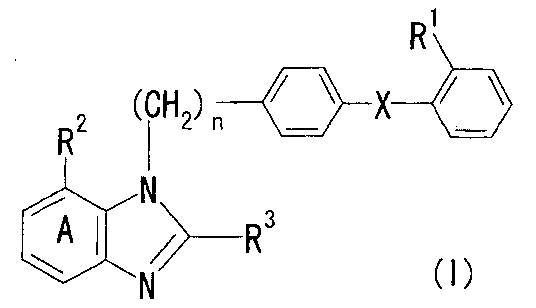 wherein R¹ denotes a group which can form an anion or group which can be converted into an anion, X denotes that a phenylene group and a phenyl group are bound directly or via a spacer having no more than 2 of atom chains, n denotes an integer of 1 or 2, a ring A denotes a benzene ring optionally further having a substituent, R² denotes a group which can form an anion or a group which can be converted into the group, and R³ denotes a hydrocarbon residue which may be bound via a hetero atom and may have a substituent (preferably hydrocarbon residue which may have a substituent and is bound via an oxygen atom) or a salt thereof is preferably used.

As the "optionally substituted nitrogen-containing heterocycle" denoted by a ring B, an optionally substituted nitrogen-containing aromatic heterocycle is preferable. In addition, as the "optionally substituted nitrogen-heterocycle" represented by a ring B, an optionally substituted 5 to 6-membered nitrogen-containing heterocycle is preferable and, inter alia, an optionally substituted 5 to 6 membered nitrogen-containing aromatic heterocycle (e.g. an imidazole ring which may be fused with an optionally substituted aromatic ring such as a benzene ring (which may have the same substituents as those exemplified as the substituent for a ring A described later) and may have a substituent; specifically, an imidazole ring optionally having a substituent, a benzimidazole ring optionally having a substituent) is preferable. As a substituent which may be possessed by the "nitrogen-containing heterocycle" in the "optionally substituted nitrogen-containing heterocycle" denoted by ring B, there are the same substituents as those exemplified as the substituent for a ring A described later.

In the above formulas, examples of a group which can form an anion (a group having a hydrogen atom which can be liberated as a proton) as R¹ include (1) a carboxyl group, (2) a tetrazolyl group, (3) a trifluoromethanesulfonic acid amide group (-NHSO₂CF₃), (4) a phosphoric acid group, (5) a sulfonic acid group, and (6) 5 to 7-membered (preferably 5 to 6-membered) monocyclic optionally substituted heterocyclic residue containing 1 or 2 or more of N, S and O.

Examples of the "5 to 7-membered (preferably 5 to 6-membered) monocyclic optionally substituted heterocyclic residue containing 1 or 2 or more of N, S and O" include 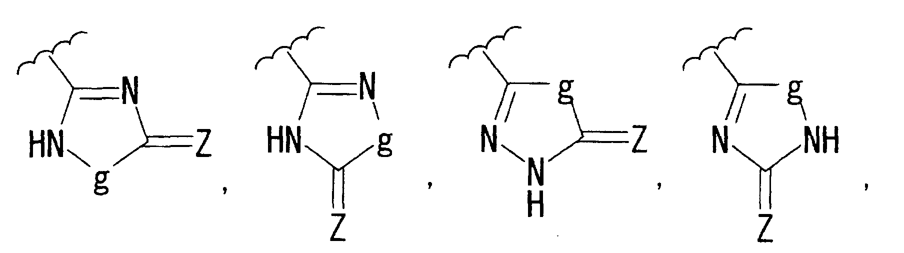 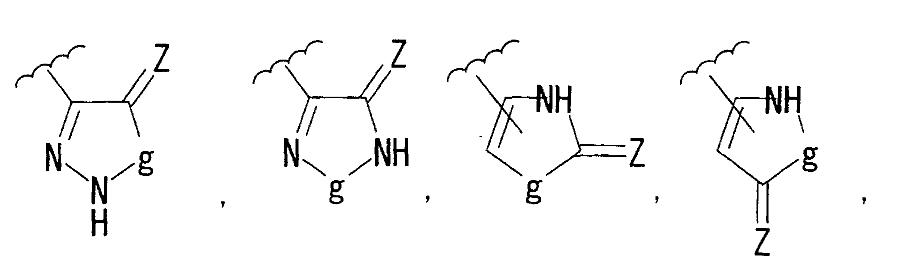 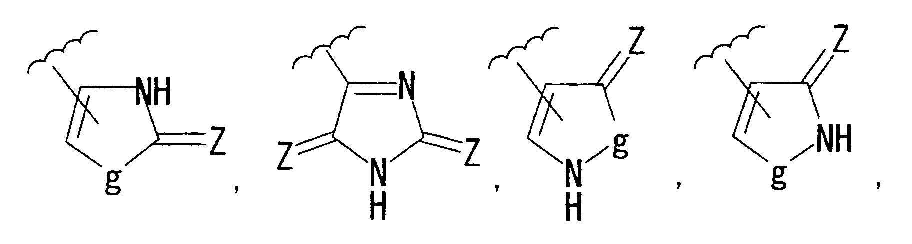 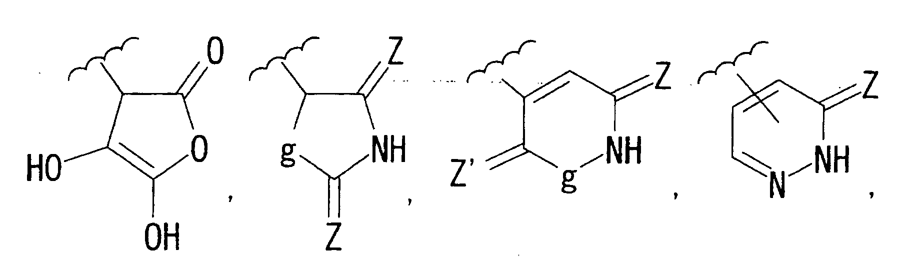 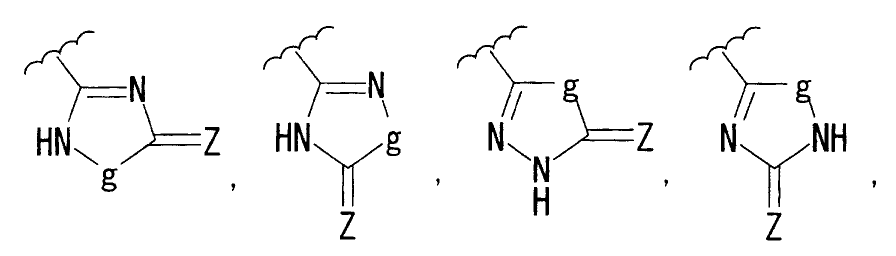 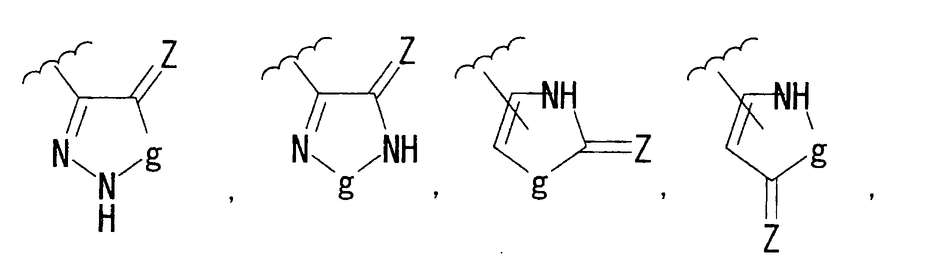 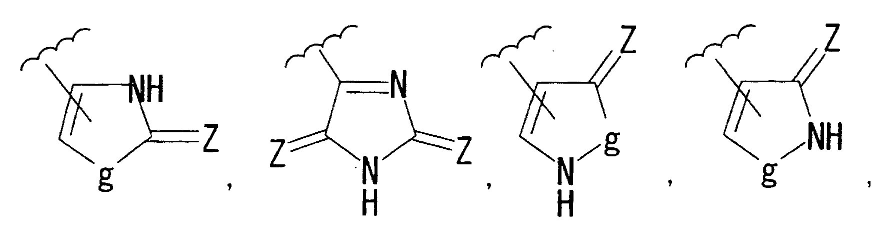 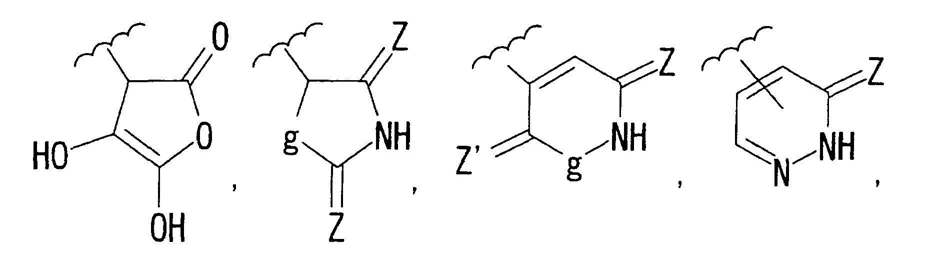 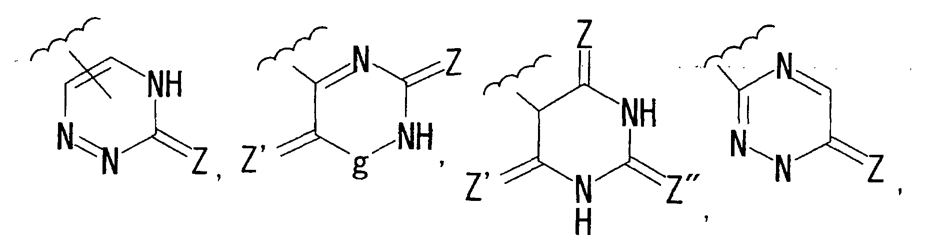 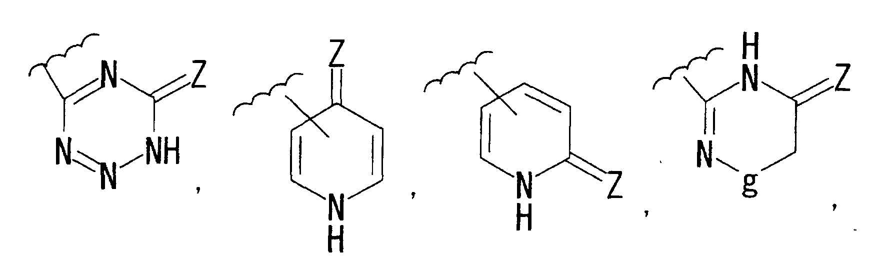 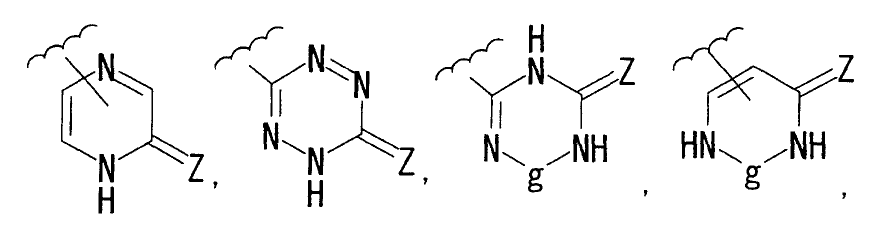 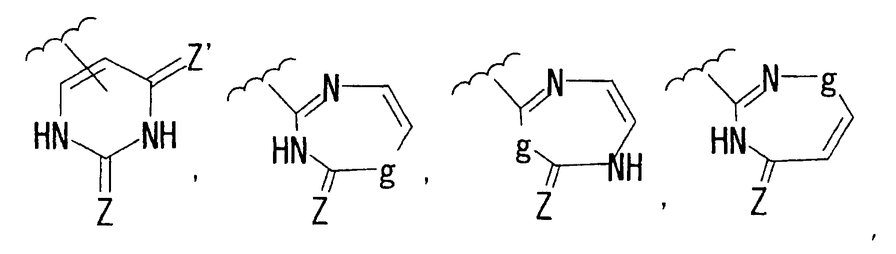 etc. The bond between the heterocyclic residue represented by R¹ and the phenyl group to be bound with the heterocyclic residue includes not only the aforementioned carbon-carbon binding but also binding via one of a plurality of nitrogen atoms when g in the above formula denotes -NH- or the like. For example, when R¹ is represented by 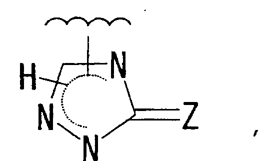 specifically, there are 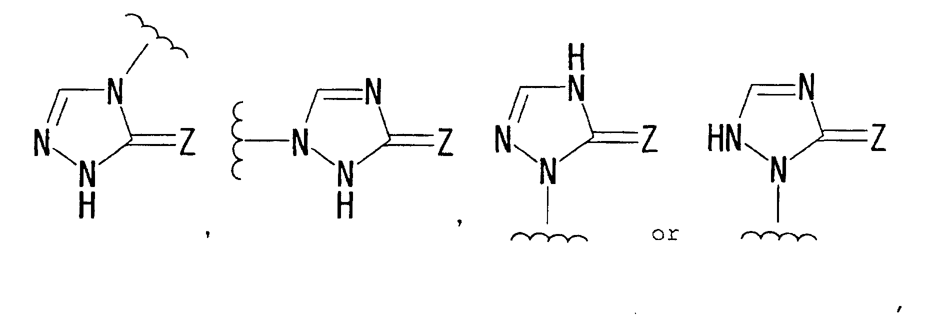 respectively. Other examples of binding via a nitrogen atom include 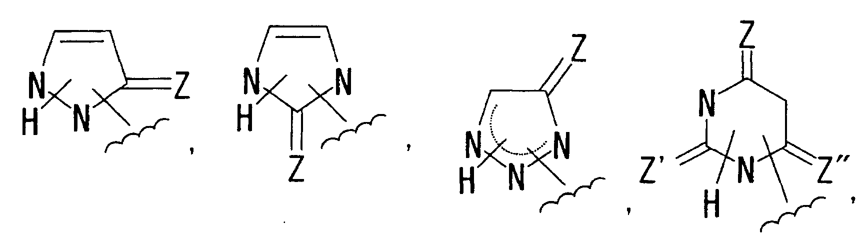 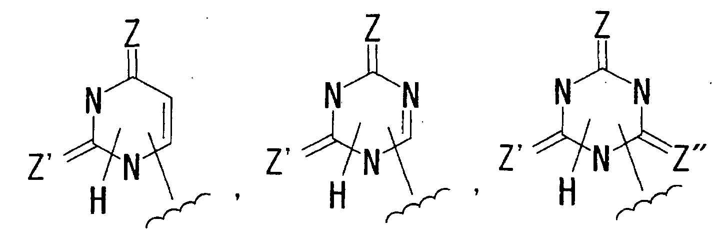 etc.

In the above formula, g denotes -CH₂-, -NH-, -O- or -S(O)ₘ-, >=Z, >=Z' and >=Z" denote a carbonyl group, a thiocarbonyl group or an optionally oxidized sulfur atom (e.g. S, S(O), S(O)₂ etc.) (preferably carbonyl or thiocarbonyl group, more preferably carbonyl group), and m denotes an integer of o, 1 or 2.

As a heterocyclic residue represented by R¹, for example, a group having an -NH- group or an -OH- group as a proton donor and a carbonyl group, a thiocarbonyl group or a sulfinyl group as a proton acceptor at the same time, such as an oxadiazolone ring, a oxadiazolothione ring or a thiadiazolone ring is preferable. In addition, a heterocyclic residue denoted by R¹ may form a fused ring by binding with a cyclic substituent and, as a heterocyclic residue represented by R¹, 5 or 6-membered ring residue is preferable, and 5-membered ring residue is more preferable.

As a heterocyclic residue represented by R¹, a group represented by the formula; 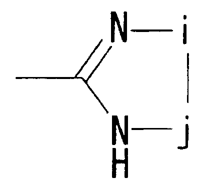 wherein i denotes -O- or -S-, j denotes >=O, >=S or >=S (O)ₘ, and m is as defined above (inter alia, 2,5-dihydro-5-oxo-1,2,4-oxadiazol-3-yl, 2,5-dihydro-5-thioxo-1,2,4-oxadizole-3-yl, 2,5-dihydro-5-oxo-1,2,4-thiadiazole-3-yl, among them, 2,5-dihydro-5-oxo-1, 2, 4-oxadiazol-3-yl) is preferable.

In addition, the heterocyclic residue (R¹) has tautomers as described below. For example, when Z=O and g=O in, 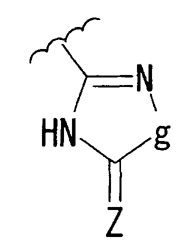 three tautomers of a', b' and c' are present as described by: 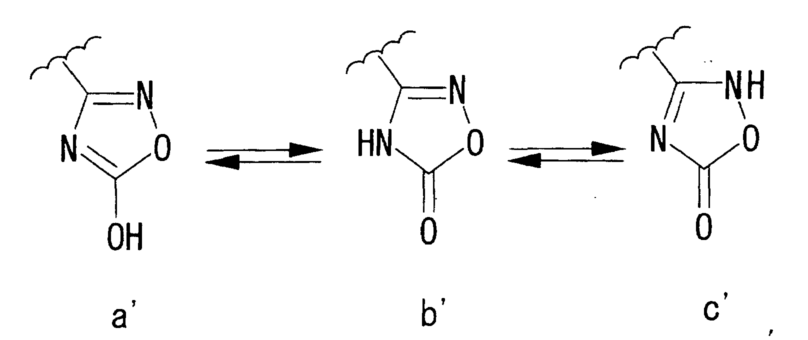 and a heterocyclic residue represented by: 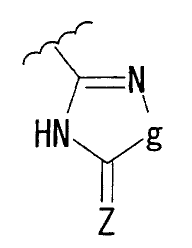 includes all of the aforementioned a', b' and c'.

A group which may form an anion as R¹ may be protected with a lower (C₁₋₄) alkyl group or an acyl group (e.g. lower (C₂₋₅) alkanoyl, benzoyl etc.), each group optionally being substituted at a replaceable position.

Examples of an optionally substituted lower (C₁₋₄)alkyl group include (1) a lower (C₁₋₄)alkyl group optionally substituted with 1 to 3 phenyl group(s) optionally having halogen atom, nitro, lower (C₁₋₄)alkyl and lower (C₁₋₄)alkoxy (e.g. methyl, triphenylmethyl, p-methoxybenzyl, p-nitrobenzyl etc.), (2) a lower (C₁₋₄)alkoxy-lower (C₁₋₄)alkyl group (e.g. methoxymethyl, ethoxymethyl etc.), and (3) a group represented by the formula -CH(R⁴)-OCOR⁵ [wherein R⁴ denotes (a) hydrogen, (b) a straight or branched lower alkyl group having a carbon number of 1 to 6 (e.g. methyl, ethyl, n-propyl, isopropyl, n-butyl, isobutyl, t-butyl, n-pentyl, isopentyl, neopentyl etc.), (c) a straight or branched lower alkenyl group having a carbon number of 2 to 6 or (d) a cycloalkyl group having a carbon number of 3 to 8 (e.g. cyclopentyl, cyclohexyl, cycloheptyl etc.) and R⁵ denotes (a) a straight or branched lower alkyl group having a carbon number of 1 to 6 (e.g. methyl, ethyl, n-propyl, isopropyl, n-butyl, isobutyl, sec-butyl, t-butyl, n-pentyl, isopentyl, neopentyl etc.), (b) a straight or branched lower alkenyl group having a carbon number of 2 to 6, (c) a lower alkyl group having a carbon number of 1 to 3 substituted with a cycloalkyl group having a carbon number of 3 to 8 (e.g. cyclopentyl, cyclohexyl, cycloheptyl etc.) or an optionally substituted aryl group (e.g. phenyl or naphthyl group optionally having halogen atom, nitro, lower (C₁₋₄)alkyl, lower (C₁₋₄)alkoxy etc.) (e.g. benzyl, p-chlorobenzyl, phenethyl, cyclopentylmethyl, cyclohexylmethyl etc.), (d) a lower alkenyl group having a carbon number of 2 to 3 substituted with cycloalkyl having a carbon number of 3 to 8 or an optionally substituted aryl group (e.g. phenyl or naphthyl group optionally having halogen atom, nitro, lower (C₁₋₄)alkyl, lower (C₁₋₄)alkoxy etc.) (e.g. groups having an alkenyl part such as vinyl, propenyl, allyl, isopropenyl etc. such as cinnamyl etc.), (e) an optionally substituted aryl group (e.g. phenyl or naphthyl group optionally having halogen atom, nitro, lower (C₁₋₄)alkyl, lower (C₁₋₄)alkoxy etc. such as phenyl, p-tolyl, naphthyl etc.), (f) a straight or branched lower alkoxy group having a carbon number of 1 to 6 (e.g. methoxy, ethoxy, n-propoxy, isopropoxy, n-butoxy, isobutoxy, sec-butoxy, t-butoxy, n-pentyloxy, isopentyloxy, neopentyloxy etc.), (g) a straight or branched lower alkenyloxy group having a carbon number of 2 to 8 (e.g. allyloxy, isobutenyloxy etc.), (h) a cycloalkyloxy group having a carbon number of 3 to 8 (e.g. cyclopentyloxy, cyclohexyloxy, cycloheptyloxy etc.), (i) a lower alkoxy group having a carbon number of 1 to 3 substituted with cycloalkyl having a carbon number of 3 to 8 (e.g. cyclopentyl, cyclohexyl, cycloheptyl etc.) or an optionally substituted aryl group (e.g. phenyl or naphthyl group optionally having halogen atom, nitro, lower (C₁₋₄)alkyl, lower (C₁₋₄)alkoxy) (e.g. groups having an alkoxy part such as methoxy, ethoxy, n-propoxy, isopropoxy etc. such as benzyloxy, phenethyloxy, cyclopentylmethoxy, cyclohexylmethoxy etc.), (j) a lower alkenyl oxy group having a carbon number of 2 to 3 substituted with cycloalkyl having a carbon number of 3 to 8 (e.g. cyclopentyl, cyclohexyl, cycloheptyl etc.) or an optionally substituted aryl group (e.g. phenyl or naphthyl group optionally having halogen atom, nitro, lower (C₁₋₄) alkyl, lower (C₁₋₄)alkoxy) (e.g. groups having an alkenyloxy part such as vinyloxy, propenyloxy, allyloxy, isopropenyloxy etc. such as cinnamyloxy etc.) or (k) an optionally substituted aryloxy group (e.g. phenoxy or naphthoxy group optionally having halogen atom, nitro, lower (C₁₋₄)alkyl, lower (C₁₋₄)alkoxy etc. such as phenoxy, p-nitrophenoxy, naphthoxy etc.)].

In addition, a group which may form an anion as R¹ may have a substituent such as an optionally substituted lower (C₁₋₄)alkyl group (examples thereof include the same "optionally substituted lower (C₁₋₄)alkyl group" as that exemplified as a protecting group for the aforementioned group which can form an anion as R¹) , halogen atom, nitro, cyano, lower (C₁₋₄)alkoxy, and amino optionally substituted with 1 to 2 lower (C₁₋₄)alkyl(s), at a replaceable place, in addition to a protecting group such as the aforementioned optionally substituted lower (C₁₋₄)alkyl group and acyl group (e.g. lower (C₂₋₅)alkanoyl, benzoyl etc.).

In the aforementioned formulas, a group which can be converted into a group which can form an anion (a group having a hydrogen atom which can be liberated as a proton) as R¹ may be a group which can be converted into a group which can form an anion (so-called prodrug) under the biological, that is, physiological conditions (e.g. a reaction in a living body such as oxidation, reduction or hydrolysis by an enzyme in a living body), or may be a group which can be converted into a group which can form an anion represented by R¹ by a chemical reaction (so-called synthetic intermediate) such as (1) a carboxyl group, (2) a tetrazolyl group, (3) a trifluoromethanesulfonic acid amide group (-NHSO₂CF₃), (4) a phosphoric acid group, (5) a sulfonic acid group, and (6) an optionally substituted 5 to 7-membered (preferably 5 or 6-membered) monocyclic heterocyclic residue containing 1 or 2 or more of N, S and O, each being protected with cyano, a N-hydroxycarbamimidoyl group (-C(=N-OH)-NH₂), or an optionally substituted lower (C₁₋₄)alkyl group or acyl group.

As R¹, carboxyl, tetrazolyl or 2,5-dihydro-5-oxo-1,2,4-oxadiazol-3-yl (preferably tetrazolyl) optionally protected with an optionally substituted lower (C₁₋₄)alkyl (e.g. methyl, triphenylmethyl, methoxymethyl, ethoxymethyl, p-methoxybenzyl, p-nitrobenzyl etc.) or an acyl group (e.g. lower (C₂₋₅)alkanoyl, benzoyl etc.), or cyano, or N-hydroxycarbamimidoyl (preferably cyano) is preferable and, inter alia, cyano is preferably used.

In the above formulas, X denotes that adjacent phenylene group and phenyl group are bound directly or via no more than 2 of atom chains (preferably direct binding) and, as a spacer having no more than 2 of atom chains, any divalent chains in which the number of atoms constituting a straight part is 1 or 2 may be used, and a spacer may have a side chain. Specifically, there are lower (C₁₋₄)alkylene, -CO-, -O-, -S-, -NH-, -CO-NH-, -O-CH₂-, -S-CH₂- and -CH=CH- in which the number of atoms constituting a straight part is 1 or 2.

In the above formulas, n denotes an integer of 1 or 2 (preferably 1).

In the above formulas, a ring A denotes a benzene ring optionally having an substituent in addition to a substituent R², and examples of the substituent include (1) halogen (e.g. F, Cl, Br etc.), (2) cyano, (3) nitro, (4) optionally substituted lower (C₁₋₄)alkyl, (5) lower (C₁₋₄)alkoxy, (6) optionally substituted amino group (e.g. amino, N-lower (C₁₋₄)alkylamino (e.g. methylamino etc.), N, N-di-lower (C₁₋₄)alkylamino (e.g. dimethylamino etc.), N-arylamino (e.g. phenylamino etc.), alicyclic amino (e.g. morpholino, piperidino, piperazino, N-phenylpiperazino etc.) etc.) (7) a group represented by the formula (-CO-D' [wherein D' denotes a hydroxyl group, or lower (C₁₋₄)alkoxy in which an alkyl part may be substituted with hydroxyl group, lower (C₁₋₄)alkoxy, lower (C₂₋₆)alkanoyloxy (e.g. acetoxy, pivaloyloxy etc.), lower (C₁₋₆)alkoxycarbonyloxy (e.g. methoxycarbonyloxy, ethoxycarbonyloxy etc.) or lower (C₃₋₆)cycloalkoxycarbonyloxy (e.g. cyclohexyloxycarbonyloxy etc.)], and (8) tetrazolyl, a trifluoromethanesulfonic acid amide group, a phosphoric acid group and a sulfonic acid group which may be protected with an optionally substituted lower (C₁₋₄)alkyl (examples thereof include the same "optionally substituted lower (C₁₋₄)alkyl group" as that exemplified as a protecting group for the aforementioned group which can form an anion as R¹) or acyl (e.g. lower (C₂₋₅)alkanoyl, benzoyl etc.).

These substituents may replace 1 to 2 replaceable position on a benzene ring at the same time and, as a substituent which is further possessed by a ring A in addition to a substituent R², an optionally substituted lower (C₁₋₄)alkyl (e.g. lower (C₁₋₄)alkyl optionally substituted with hydroxyl group, carboxyl group, halogen etc.), and halogen are preferable, and it is more preferable that a ring A does not have a substituent in addition to a substituent R².

In the above formulas, examples of a group which can form an anion (a group having a hydrogen atom which can be liberated as a proton) as R² include (1) an optionally esterified or amidated carboxyl group, (2) a tetrazolyl group, (3) a trifluoromethanesulfonic acid amide group (-NHSO₂CF₃), (4) a phosphoric group and (5) a sulfonic acid group, and these groups may be protected with an optionally substituted lower alkyl group (examples thereof include the same "optionally substituted lower (C₁₋₄)alkyl group" as that exemplified as a protecting group for the aforementioned group which can form an anion as R¹) or an acyl group (e.g. lower (C₂₋₅)alkanoyl benzyl etc.), and any groups may be used as far as they are a group which can form an anion under the biological, that is, physiological conditions (a reaction in a living body such as oxidation, reduction and hydrolysis by an enzyme in a living body) or chemically, or a group which can be converted into the group.

Examples of an optionally esterified or amidated carboxyl as R² include a group represented by the formula -CO-D [wherein D denotes (1) hydroxyl group, (2) optionally substituted amino (e.g. amino, N-lower (C₁₋₄)alkylamino, N,N-di-lower (C₁₋₄)alkylamino etc.) or (3) optionally substituted alkoxy {e.g. (i) a lower (C₁₋₆)alkoxy group in which an alkyl part may be substituted with hydroxyl group, optionally substituted amino (e.g. amino, N-lower (C₁₋₄)alkylamino, N,N-di-lower (C₁₋₄)alkylamino, piperidino, morpholino etc.), halogen, lower (C₁₋₆)alkoxy, lower (C₁₋₆)alkylthio, lower (C₃₋₈)cycloalkoxy or optionally substituted dioxolenyl (e.g. 5-methyl-2-oxo-1,3-dioxolen-4-yl etc.), or (ii) a group represented by the formula -O-CH(R⁶)-OCOR⁷ [wherein R⁶ denotes (a) hydrogen, (b) a straight or branched lower alkyl group having a carbon number of 1 to 6 (e.g. methyl, ethyl, n-propyl, isopropyl, n-butyl, isobutyl, t-butyl, n-pentyl, isopentyl, neopentyl etc.), (c) a straight or branched lower alkenyl group having a carbon number of 2 to 6 or (d) a cycloalkyl group having a carbon number of 3 to 8 (e.g. cyclopentyl, cyclohexyl, cycloheptyl etc.) and R⁷ denotes (a) a straight or branched lower alkyl group having a carbon number of 1 to 6 (e.g. methyl, ethyl, n-propyl, isopropyl, n-butyl, isobutyl, sec-butyl, t-butyl, n-pentyl, isopentyl, neopentyl etc.), (b) a straight or branched lower alkenyl group having a carbon number of 2 to 6, (c) a lower alkyl group having a carbon number of 1 to 3 substituted with a cycloalkyl group having a carbon number of 3 to 8 (e.g. cyclopentyl, cyclohexyl, cycloheptyl etc.) or an optionally substituted aryl group (e.g. phenyl or naphthyl group optionally having halogen atom, nitoro, lower (C₁₋₄)alkyl and lower (C₁₋₄)alkoxy) (e.g. benzyl, p-chlorobenzyl, phenethyl, cyclopentylmethyl, cyclohexylmethyl etc.), (d) a lower alkenyl group having a carbon number of 2 to 3 substituted with cycloalkyl having a carbon number of 3 to 8 or optionally substituted aryl group (e.g, phenyl or naphthyl group optionally having halogen atom, nitro, lower (C₁₋₄)alkyl, and (C₁₋₄)alkoxy) (e.g. groups having an alkenyl part such as vinyl, propenyl, allyl, isopropenyl etc. such as cinnamyl etc.), (e) an optionally substituted aryl group (e.g. phenyl or naphthyl group optionally having halogen atom, nitro, lower (C₁₋₄)alkyl, lower (C₁₋₄)alkoxy etc. such as phenyl, p-tolyl, naphthyl etc.), (f) a straight or branched lower alkoxy group having a carbon number of 1 to 6 (e.g. methoxy, ethoxy, n-propoxy, isopropoxy, n-butoxy, isobutoxy, sec-butoxy, t-butoxy, n-pentyloxy, isopentyloxy, neopentyloxy etc.), (g) a straight or branched lower alkenyl oxy group having a carbon number of 2 to 8 (e.g. allyloxy, isobutenyloxy etc,), (h) a cycloalkyloxy group having a carbon number of 3 to 8 (e.g. cyclopentyloxy, cyclohexyloxy, cycloheptyloxy etc.), (i) a lower alkoxy group having a carbon number of 1 to 3 substituted with cycloalkyl having a carbon number of 3 to 8 (e.g. cyclopentyl, cyclohexyl, cycloheptyl etc.) or an optionally substitute aryl group (e.g. phenyl or naphthyl group optionally having halogen atom, nitro, lower (C₁₋₄)alkyl lower (C₁₋₄)alkoxy etc.) (e.g. groups having an alkoxy group such as methoxy, ethoxy, n-propoxy, isopropoxy etc. such as benzyloxy, phenethyloxy, cyclopentylmethoxy, cyclohexylmethoxy etc.) (j) a lower alkenyloxy group having a carbon number of 2 to 3 substituted with cycloalkyl having a carbon number of 3 to 8 (e.g. cyclopentyl, cyclohexyl, cycloheptyl etc.) or an optionally substituted aryl group (e.g. phenyl or naphthyl group optionally having halogen atom, nitro, lower (C₁₋₄)alkyl, lower (C₁₋₄)alkoxy etc.) (e.g. groups having an alkenyloxy part such as vinyloxy, propenyloxy, allyloxy, isopropenyloxy etc. such as cinnamyloxy etc.) or (k) an optionally substituted aryloxy group (e.g. phenoxy or naphthoxy group optionally having halogen atom, nitro, lower (C₁₋₄)alkyl, lower (C₁₋₄)alkoxy etc. such as phenoxy, p-nitrophenoxy, naphthoxy etc.)]}].

As R², optionally esterified carboxyl is preferable, and examples thereof include -COOH and a salt thereof, -COOMe, -COOEt, -COOtBu, -COOPr, pivaloyloxymethoxycarbonyl, 1-(cyclohexyloxycarbonyloxy)ethoxycarbonyl, 5-methyl-2-oxo-1,3-dioxolen-4-ylmethoxycarbonyl, acetoxymethoxycarbonyl, propionyloxymethoxycarbonyl, n-butyloxymethoxycarbonyl, isobutyloxymethoxycarbonyl, 1-(ethoxycarbonyloxy)ethoxycarbonyl, 1-(acetoxy)ethoxycarbonyl, 1-(isobutyloxy)ethoxycarbonyl, cyclohexylcarbonyloxymethoxycarbonyl, benzoyloxymethoxycarbonyl, cinnamyloxycarbonyl and cyclopentylcarbonyloxymethoxycarbonyl, and any groups may be used as far as they are a group which can form an anion (e.g. COO⁻, a derivative thereof etc.) under the biological, that is, physiological conditions (e.g. a reaction in a living body such as oxidation, reduction and hydrolysis by an enzyme in living body) or chemically, or a group which can be converted into the group, or it may be a carboxyl group, or a prodrug thereof.

As the R², a group represented by the formula -CO-D [wherein D denotes (1) a hydroxyl group or (2) lower (C₁₋₄)alkoxy in which an alkyl part may be substituted with hydroxyl group, amino, halogen, lower (C₂₋₆)alkanoyloxy (e.g. acetoxy, pivaloyloxy etc.), lower (C₃₋₈)cycloalkanoyloxy, lower (C₁₋₆)alkoxycarbonyloxy (e.g. methoxycarbonyloxy, ethoxycarbonyloxy etc.), lower (C₃₋₈)cycloalkoxycarbonyloxy (e.g. cyclohexyloxycarbonyloxy etc.), lower (C₁₋₄)alkoxy or lower (C₃₋₈)cycloalkoxy] is preferable and, inter alia, carboxyl esterified with lower (C₁₋₄)alkyl (preferably methyl or ethyl) is preferable.

In the above formulas, examples of the "hydrocarbon residue" in the "hydrocarbon residue which may be bound via a hetero atom and has a substituent" represented by R³ include (1) an alkyl group, (2) an alkenyl group, (3) an alkynyl group, (4) a cycloalkyl group, (5) an aryl group and (6) an aralkyl group and, inter alia, an alkyl group, an alkenyl group and a cycloalkyl group are preferable.

The (1) alkyl group may be a straight or branched lower alkyl group having a carbon number of around 1 to 8, and examples thereof include methyl, ethyl, propyl, isopropyl, butyl, isobutyl, sec-butyl, t-butyl, pentyl, i-pentyl, hexyl, heptyl and octyl.

The (2) alkenyl group may be a straight or branched lower alkenyl group having a carbon number of around 2 to 8, and examples thereof include vinyl, propenyl, 2-butenyl, 3-butenyl, isobutenyl and 2-octenyl.

The (3) alkynyl group may be a straight or branched lower alkynyl group having a carbon number of around 2 to 8, and examples thereof include ethynyl, 2-propynyl, 2-butynyl, 2-penthynyl and 2-octynyl.

Examples of the (4) cycloalkyl group include lower cycloalkyl having a carbon number of around 3 to 6, for example, cyclopropyl, cyclobutyl, cyclopentyl and cyclohexyl.

The aforementioned alkyl group, alkenyl group, alkynyl group or cycloalkyl group may be substituted with a hydroxyl group, an optionally substituted amino group (e.g. amino, N-lower (C₁₋₄)alkylamino, N,N-di-lower (C₁₋₄)alkylamino etc.), halogen, lower (C₁₋₄)alkoxy group, or lower (C₁₋₄)alkylthio group.

Examples of the (5) aralkyl group include phenyl-lower (C₁₋₄)alkyl such as benzyl and phenethyl, and examples of the (6) aryl group include phenyl.
The aforementioned aralkyl group or aryl group may have halogen (e.g. F, Cl, Br etc.), nitro, optionally substituted amino group (e.g. amino, N-lower (C₁₋₄)alkylamino, N,N-di-lower (C₁₋₄)alkylamino etc.), lower (C₁₋₄)alkoxy (e.g. methoxy, ethoxy etc.), lower (C₁₋₄)alkylthio (e.g. methylthio, ethylthio etc.), or lower (C₁₋₄)alkyl (e.g. methyl, ethyl etc.) at an arbitrary position on a benzene ring.

Among the forgoing, as the "hydrocarbon residue" in the "hydrocarbon residue which may be bound via a hetero atom and has a substituent" represented by R³, an optionally substituted alkyl or alkenyl (e.g. lower (C₁₋₅)alkyl or lower (C₂₋₅)alkenyl group optionally substituted with hydroxyl group, amino group, halogen or lower (C₁₋₄)alkoxy group) is preferable and, inter alia, lower (C₁₋₅)alkyl (more preferably ethyl) is preferable.

Examples of the "hetero atom" in the "hydrocarbon residue which may be bound via a hetero atom and has a substituent" represented by R³ include -O-, -S(O)ₘ- [m denotes an integer of 0 to 2], and -NR'- [R' denotes a hydrogen atom or lower (C₁₋₄)alkyl] and, inter alia, -O- is preferably used.

Among the foregoing, as R³, a lower (C₁₋₅)alkyl group and lower (C₂₋₅)alkenyl group which may be bound via -O-, -S(O)ₘ- [m denotes an integer of 0 to 2] or -NR'- [R' denotes hydrogen atom or lower (C₁₋₄)alkyl] and may be substituted with an substituent selected from a hydroxyl group, an amino group, a halogen and a lower (C₁₋₄)alkoxy group are preferable and, inter alia, lower (C₁₋₅)alkyl and lower (C₁₋₅)alkoxy (more preferable ethoxy) are preferable.

Among compounds having the angiotensin II antagonistic activity represented by the formula (I), a benzimidazole-7-carboxylic acid derivative represented by the formula (I'): 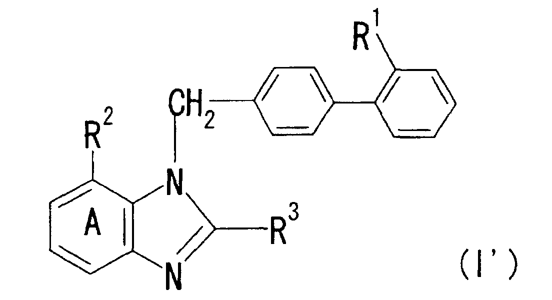 (wherein R¹ denotes (1) a carboxyl group, (2) a tetrazolyl group or (3) a group represented by the formula: 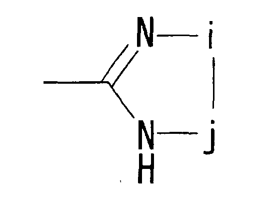 [wherein i denote -O- or -S-, j denotes >=O, >=S or >=S (O)ₘ, and m is as defined above], a ring A denotes a benzene ring (preferably a benzene ring having no substituent other than a substituent R² optionally substituted with optionally substituted lower (C₁₋₄)alkyl (e.g. lower (C₁₋₄)alkyl optionally substituted with hydroxyl group, carboxyl group or halogen) or halogen in addition to a substituent R², R² denotes a group represented by the formula-CO-D [wherein D denotes (1) hydroxyl group or (2) lower (C₁₋₄)alkoxy in which an alkyl part may be substituted with hydroxyl group, amino, halogen, lower (C₂₋₆)alkanoyloxy (e.g. acetoxy, pivaloyloxy etc.), lower (C₃₋₈)cycloalkanoyloxy, lower (C_{1- 6})alkoxycarbonyloxy (e.g. methoxycarbonyloxy, ethoxycarbonyloxy etc.), lower (C₃₋₈)cycloalkoxycarbonyloxy (e.g. cyclohexyloxycarbonyloxy etc.), lower (C₁₋₄)alkoxy or lower (C₃₋₈)cycloalkoxy], R³ denotes lower (C₁₋₅)alkyl or lower (C₂₋₅)alkenyl group (preferably lower (C₁₋₅)alkyl or lower (C₁₋₅)alkoxy; more preferably ethoxy) which may be bound via -O-, -S(O)ₘ- [m denotes an integer of 0 to 2] or -NR'- [R' denotes hydrogen atom or lower (C₁₋₄)alkyl] and may be substituted with a substituent selected from a hydroxyl group, an amino group, a halogen and lower (C_{1- 4})alkoxy group]), or a pharmacologically acceptable salt is preferable and, inter alia, 2-ethoxy-1-[[2'-(1H-tetrazol-5-yl)biphenyl-4-yl]methyl]benzimidazole-7-carboxylic acid [Candesartan], 1-(cyclohexyloxycarbonyloxy)ethyl 2-ethoxy-1-[[2'-(1H-tetrazol-5-yl)biphenyl-4-yl]methyl]benzimidazole-7-carboxylate [Candesartan cilexetil], pivaloyloxymethyl 2-ethoxy-1-[[2'-(1H-tetrazol-5-yl)biphenyl-4-yl]methyl]benzimidazol-7-carboxylate, and 2-ethoxy-1-[[2'-(5-oxo-2, 5-dihydro-1,2,4-oxadiazol-3-yl)biphenyl-4-yl]methyl]-1H-benzimidazole-7-carboxylic acid or a salt thereof are preferable.

Here, the aforementioned 2-ethoxy-1-[[2'-(5-oxo-2,5-dihydro-1,2,4-oxadiazol-3-yl)biphenyl-4-yl]methyl-1H-benzimidazole-7-carboxylic acid is represented -by the formula: 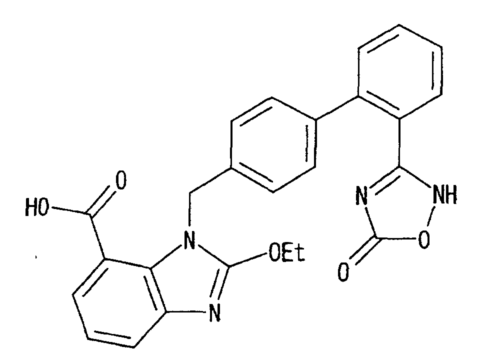 and is called as 2-ethoxy-1-[[2'-(2,5-dihydro-5-oxo-1,2,4-oxadiazol-3-yl)biphenyl-4-yl]methyl]benzimidazol-7-carboxylic acid in some cases, but those have the chemical-structurally same meaning.

The aforementioned benzimidazole derivative can be synthesized by the known method described in, for example, EP-425921, EP-459136, EP-553879, EP-578125, EP-520423 and EP-668272 or a similar method. When Candesartan cilexetil is used, it is better to use the stable C-type crystal described in EP-459136.

A compound having the angiotensin II antagonistic activity or a prodrug thereof used in the present invention may be itself or a pharmacologically acceptable salt thereof. When the compound having the angiotensin II antagonistic activity has an acidic group such as a carboxyl group and the like, examples of such the salt include salts with inorganic bases (e.g. alkali metal such as sodium, potassium etc., alkaline earth metal such as calcium, magnesium etc., transition metal such as zinc, iron, copper etc.) or salts with organic bases (e.g. organic amines such as trimethylamine, triethylamine, pyridine, picoline, ethanolamine, diethanolamine, triethanolamine, dicyclohexylamine and N,N'-dibenzylethylenediamine, basic amino acids such as alginine, lysine and ornithine).

When a compound having the angiotensin II antagonistic activity has a basic group such as an amino group and the like, examples are salts with inorganic acids or organic acids (e.g. hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid etc.) or acidic amino acids such as aspartic acid and glutamic acid.

A prodrug of a compound having the angiotensin II antagonistic activity used in the present invention [hereinafter, referred to as All antagonistic compound in some cases] refers to a compound which is converted into the All antagonistic compound by a reaction with an enzyme or stomach acid under the physiological conditions in a living body, that is, a compound which undergoes enzymatic oxidation, reduction or hydrolysis and is changed into the All antagonistic compound, or a compound which undergoes hydrolysis by stomach acid and is changed into the All antagonistic compound. Examples of a prodrug of the All antagonistic compound include a compound in which an amino group of the All antagonistic compound is acylated, alkylated or phosphorylated (e.g. a compound in which an amino group of the All antagonistic compound is eicosanoylated, alanylated, pentylaminocarbonized, (5-methyl-2-oxo-1,3-dioxolen-4-yl)methoxycarbonized, tetrahydrofuranylated, pyrrolidylmethylated, pivaloyloxymethylated or tert-butylated); a compound in which a hydroxyl group of the All antagonistic compound is acylated, alkylated, phosphorylated or borated (e.g. a compound in which a hydroxyl group of the All antagonistic compound is acetylated, palmitoylated, propanoylated, pivaloylated, succinylated, fumarylated, alanylated or dimethylaminomethylcarbonized); a compound in which a carboxyl group of the All antagonistic compound is esterified or amidated (e.g. a compound in which a carboxyl group of the All antagonistic compound is ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonyloxyethylesterified, or methylamidated); and the like. These compounds can be prepared from the All antagonistic compound by the known per se method.

Alternatively, a prodrug of the All antagonistic compound may be a prodrug which is changed into the AII antagonistic compound under the physiological conditions described in "Development of Medicaments", vol.7, Molecular Design, pp 163-198 published by Hirokawashoten in 1990.

The All antagonistic compound may be either of a hydrate or a non-hydrate.

The All antagonistic compound, in particular, a compound represented by the formula (I) and a pharmaceutically acceptable salt thereof are low toxic, and can be used as an analgesic agent or an antiinflammatory agent to a mammal (e.g. human, mouse, rat, rabbit, dog, cat, cow, pig, monkey etc.), as they are, or by mixing with a pharmaceutically acceptable carrier to formulate into a pharmaceutical composition.

Herein, as a pharmaceutically acceptable carrier, various organic or inorganic carrier materials which are conventional as a pharmaceutical material are used, and is incorporated as an excipient, a lubricant, a binder or a disintegrating agent in a solid preparation; as a solvent, a solubilizer, a suspending agent, an isotonic, a buffer or a soothing agent in a liquid preparation. If necessary, pharmaceutical additives such as a preservative, an antioxidant, a colorant and a sweetener may be used.

Preferable examples of an excipient include lactose, sucrose, D-mannitol, D-sorbitol, starch, gelatinized starch, dextrin, crystalline cellulose, low-substituted hydroxypropylcellulose, sodium carboxymethylcellulose, gum arabic, dextrin, pullulan, light silisic anhydride, synthetic aluminium silicate and magnesium aluminate metasilicate.

Preferable examples of a lubricant include magnesium stearate, calcium stearate, talc and colloidal silica. Examples of a binder include gelatinized starch, sucrose gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose and polyvinylpyrrolidone.

Preferable examples of a disintegrating agent include lactose, sucrose, starch, carboxymethylcellulose, potassium carboxylmethylcellulose, sodium crosscarmerose, sodium carboxymethylstarch, light silisic anhydride and low-substituted hydroxypropylcellulose.

Preferably examples of a solvent include water for injection, physiological saline, Ringer's solution, alcohol, propylene glycol, polyethylene glycol, sesame oil, corn oil, olive oil and cottonseed oil.

Preferable examples of a solubilizer include polyethylene glycol, propylene glycol, D-mannitol, trehalose, benzyl benzoate, ethanol, trisaminomethane, cholesterol, triethanolamine, sodium carbonate, sodium citrate, sodium salicylate and sodium acetate.

Preferable examples of a suspending agent include surfactants such as stearyltriethanolamine, sodium laurylsulfate, laurylaminopropionic acid, lecithin, benzalkonium chloride, benzethonium chloride and monostearic acid glycerin; hydrophilic polymers such as polyvinyl alcohol, polyvinyl pyrrolidone, sodium carboxymethylcellulose, methylcellulose, hydroxymethylcellulose, hydroxyethylcellulose and hydroxypropylcellulose; polysorbates and polyoxyethylene hydrogenated castor oil.

Examples of a preferable isotonic include sodium chloride, glycerin, D-mannitol, D-sorbitol and glucose. Preferable examples of a buffer include buffers such as phosphate, acetate, carbonate and citrate.

Examples of a soothing agent include benzyl alcohol.

Preferable examples of a preservative include paraoxybenzoic acid esters, chlorobutanol, benzyl alcohol, phenethyl alcohol, dehydroacetic acid and sorbic acid.

Preferable examples of an antioxidant include sulfite and ascorbate.

Preferable examples of a colorant include water-soluble edible tar pigments (e.g. edible pigments such as edible red Nos. 2 and 3, edible yellow Nos. 4 and 5, edible blue Nos. 1 and 2), water-insoluble lake pigments (e.g. aluminium salt of the aforementioned water-soluble tar pigments), and natural pigments (e.g. β-carotene, chlorophyll and colcothar).

Preferable examples of a sweetener include sodium saccharine, dipotassium glycyrrhizinate, aspartame and stevia.

Dosage forms of a pharmaceutical composition include oral preparations such as tablets, capsules (including soft capsules and microcapsules), granules, powders, syrups, emulsions and suspensions; parenteral preparations such as injectables (e.g. subcutaneous injectables, intravenous injectables, intramuscular injectables, intraperitoneal injectables, intravitreous injectables), eye drops, external preparations (e.g. transnasal preparations, transdermal preparations and ointments), suppositories (rectal suppositories, vaginal suppositories), pellets, eye drops and sustained-releasing preparations and these can be safely administered orally or parenterally.

Pharmaceutical compositions can be prepared by the conventional methods in pharmacy technical fields, for example, methods described in Japanese Pharmacopoeia. Specific process for preparing preparations will be described in detail below.

The content of the All antagonistic compound or a pharmacologically acceptable salt in a pharmaceutical composition is about 0.001% by weight to about 95% by weight, preferably about 0.1% by weight to about 70% by weight relative to a total amount of composition.

For example, oral preparations are prepared by adding an excipient (e.g. lactose, sucrose, starch, D-mannitol etc.), disintegrating agent (e.g. potassium carboxymethylcellulose etc.), a binder (e.g. gelatinized starch, gum arabic, carboxymethylcellulose, hydroxypropylcellulose, polyvinylpyrrolidone etc.) or a lubricant (e.g. talc, magnesium stearate, polyethylene glycol 6000 etc.) to an active ingredients, compression-molding them and, if necessary, coating a molded material using a coating base by the known per se method for the purpose of taste masking, solubility in intestine or the sustaining property.

Examples of the coating base include sugar-coated base, water-soluble film coating base, enteric film coating base, sustained-releasing film coating base and the like.

As the sugar-coated base, sucrose is used, and 1 or 2 or more selected from precipitated calcium carbonate, gelatin, gum arabic, pullulan and carnauba wax may be used jointly.

Examples of a water-soluble film coating base include cellulose series polymers such as hydroxypropylcellulose, hydroxypropylmethylcellulose, hydroxyethylcellulose and methylhydroxyethylcellulose; synthetic polymers such as polyvinylacetal diethylaminoacetate, aminoalkyl methacrylate copolymer E [Eudragit E (trade name), Rohmpharma] and polyvinylpyrrolidone; polysaccharides such as pullulan.

Examples of an enteric-soluble film coating base include cellulose series polymers such as hydroxypropylmethylcellulose phthalate, hydroxypropylmethylcellulose acetate succinate, carboxymethylethylcellulose, and cellulose acetate phthalate; acrylic acid series polymers such as methacrylic acid copolymer L [Eudragit L (trade name), Rohmpharma], methacrylic acid copolymer LD [Eudragit L-30D55 (trade mane), Rohmpharma], and methacrylic acid copolymer S [Eudragit S (trade name), Rohmpharma]; natural materials such as shellac.

Examples of the sustained-releasing film coating base include cellulose series polymers such as ethylcellulosel; acrylic acid series polymers such as aminoalkyl methacrylate copolymer (RS) [Eudragit RS (trade name) Rohmpharma], ethyl acrylate•methyl methacrylate copolymer suspension [Eudragit NE (trade name), Rohmpharma].

The aforementioned coating bases may be used by mixing 2 or more kinds of them at an appropriately ratio. In addition, upon coating, light shielding agent such as titanium oxide and iron sesquioxide may be used.

Injectables are prepared by dissolving, suspending or emulsifying an active ingredient together with a dispersing agent (e.g. polysorbate 80, polyoxyethylene hydrogenated castor oil 60, polyethylene glycol, carboxymethylcellulose, sodium arginate etc.), a preservative (e.g. methylparaben, propylparaben, benzyl alcohol, chlorobutanol, phenol etc.), an isotonic (e.g. sodium chloride, glycerin, D-mannitol, D-sorbitol, glucose etc.) in an aqueous solvent (e.g. distilled water, physiological saline, Linger's solution etc.) or an oily solvent (e.g. vegetable oil such as olive oil, sesame oil, cottonseed, corn oil etc.. propylene glycol). Upon this, additives such as a solubilizer (e.g. sodium salicylate, sodium acetate etc.), a stabilizing agent (e.g. human albumin etc.), a soothing agent (benzyl alcohol etc.) may be used.

A dose of the All antagonistic compound is different depending on a subject to be administered, an administration route, a subject disease and the symptom and, for example, when orally administered to an adult (weight 50kg), letting a one time amount of a compound represented by the formula (I) as an active ingredient or a pharmaceutically acceptable salt thereof to be usually about 0.001 to 500mg, preferably 0.1 to 50mg, it is desirable that this amount is administered once to three times a day.

The aforementioned "compound having the angiotensin II antagonistic activity", inter alia, preferably a compound represented by the formula (I), or a salt thereof, or a prodrug thereof may be applied as an analgesic agent or an antiinflammatory agent by formulating the material and a biodegradable polymer into a safe sustained -releasing preparation. Such the sustained-releasing preparation can be prepared according to the known per se process and, for example, the preparation can be prepared according to a process described in WO99/44590 gazette, WO01/60410 gazette, Japanese Patent Application No.2001-77157 and Japanese Patent Application No.2001-353757, and applied as an analgesic agent or an antiinflammatory agent.

Examples of such the sustained-releasing preparation include:
[1] a sustained-releasing preparation comprising a compound having the angiotensin II antagonistic activity (e.g. a compound represented by the formula (II)) or a salt thereof, and a biodegradable polymer
[2] the sustained-releasing preparation described in [1], wherein the biodegradable polymer is an α-hydroxy carboxylic acid polymer,
[3] the sustained-releasing preparation described in [1], wherein the α-hydroxy carboxylic acid polymer is a lactic acid-glycolic acid polymer,
[4] the sustained-releasing preparation described in [3], wherein a molar ratio of lactic acid and glycolic acid is 100/0 to 40/60,
[5] the sustained-releasing preparation described in [2], wherein a weight average molecular weight of the polymer is 3,000 to 50,000,
[6] the sustained-releasing preparation described in [1], which is for injection,
[7] the sustained-releasing preparation described in [1], which contains a multivalent metal,
[8] the sustained-releasing preparation described in [7], wherein the multivalent metal is zinc, and
[9] a sustained-releasing preparation, comprising a compound having the angiotensin II antagonistic activity (e.g. a compound represented by the formula (I)) or a salt thereof, a biodegradable polymer and a multivalent metal.

Such the sustained-releasing preparation can be prepared and used according to a process described in WO99/44590 gazette.

The sustained-releasing preparation can be administered as an injectable or an implant to muscle, subcutaneous part or organ, a transmucous preparation to nostril, rectum or uterus, or an oral preparation (e.g. capsule (e.g. solid preparation such as hard capsule, soft capsule etc.), granule, powder etc., liquid preparation such as syrup, emulsion, suspension etc.), as it is, or by formulating into various dosage forms using the sustained-releasing preparation as a raw material substance. In addition, the preparation may be administered with a jet injector.

For example, when the sustained releasing preparation is formulated into an injectable, the preparation and a dispersing agent (e.g. surfactant such as Tween 80, HCO-60 etc., polysaccharide such as sodium hyaluronate, carboxymethylcellulose, sodium arginate etc.), a preservative (e.g. methylparaben, propylparaben etc.), an isotonic (e.g. sodium chloride, mannitol, sorbitol, glucose, proline etc.) and the like are formulated into an aqueous suspension, or the preparation and a vegetable oil such as sesame oil and corn oil are dispersed into an oily suspension, whereby, a sustained-releasing injectable which can be actually used can be obtained.

When used as a suspension injectable, a particle diameter of the sustained-releasing preparation may be such that the dispersion degree and the needle-permeability are satisfied. For example, a particle diameter as an average particle diameter is in a range of about 0.1 to 300µm, preferably in a range of about 0.5 to 150µm, more preferably in a range of about 1 to 100µm.

For formulating the sustained-releasing preparation into a sterile preparation, there are a method using all process steps under the sterile condition, a method of sterilizing with a gamma-ray, and a method of adding a preservative, being not limiting.

Since the sustained-releasing preparation is low toxic, it can be used as a safe medicament for a mammal (e.g. human, cow, pig, dog, cat, mouse, rat, rabbit etc).

A dose of a sustained-releasing preparation is variously different depending on a kind and a content of a compound having the All antagonistic activity which is a basis, a dosage form, a duration time for release of a compound having the All antagonistic activity, the symptom of a disease, and a subject animal, and a dose may be such an amount that an effective amount of a compound having the All antagonistic activity lasts. When the sustained-releasing preparation is a one month preparation, a dose of a compound having the All antagonistic activity which is a basis per once can be appropriately selected from a range of about 0.01mg to 50mg/kg-weight, more preferably a range of about 0.1mg to 30mg/kg-weight per adult.

An administration time can be appropriately selected depending on a kind and a content of a compound having the All antagonistic activity which is a basis, a dosage form, a duration time of release of a compound having the All antagonistic activity, the symptom of a disease, and a subject animal, such as once per few week, once per month, once per months (e.g. 3 months, 4 months, 6 months etc.), and the like.

The analgesic agent and the antiinflammatory agent of the present invention is used to a mammal (e.g. human, mouse, rat, rabbit, dog, cat, cow, horse, pig, monkey etc.) For example, the agent of the present invention is used as an agent for preventing or treating inflammatory diseases. Examples of inflammatory diseases include inflammatory diseases due to various diseases such as arthritis (e.g. rheumatoid arthritis, osteoarthritis, rheumatoid myelitis, gouty arthritis, synovitis), asthma, allergic disease, arteriosclerosis including atherosclerosis (aneurysm, coronary sclerosis, cerebral arterial sclerosis, peripheral arterial sclerosis etc.), digestive tract disease such as inflammatory intestine disease (e.g. Crohn's disease, ulcerative colitis), diabetic complication (diabetic nerves disorder, diabetic vascular disorder), atopic dermatitis, chronic obstructive pulmonary disease, systemic lupus erythematosus, visceral inflammatory disease (nephritic, hepatitis), autoimmune hemolytic anemia, psoriasis, nervous degenerative disease (e.g. Alzheimer's disease, Parkinson's diseases, amyotrophic lateral sclerosis, AIDS encephalopathy), central nervous disorder (e.g. cerebrovascular disorder such as cerebral hemorrhage and cerebral infarct, head trauma, spinal damage, cerebral edema, multiple sclerosis), meningitis, angina, cardiac infarct, congestive cardiac failure, vascular hypertrophy or occulusion and organ disorder after intervention (transdermal coronary plasty, stent indwelling, coronary endoscope, intravascular ultrasound, intracoronary thrombolysis etc), vascular reocculusion or restenosis after bypass operation, endothelial functional disorder, other circulatory disease (intermittent claudication, obstructive peripheral circulatory disorder, obstructive arteriosclerosis, obstructive thrombotic angitis, ischemic cerebral circulatory disorder, Leiner's disease, Verger's disease), inflammatory ocular disease, inflammatory pulmonary disease (e.g. chronic pneumonia, silicosis, pulmonary sarcoidosis, pulmonary tuberculosis), endometritis, toxemia (e.g. sepsis, septic shock, endotoxin shock, gram negative sepsis, toxin shock syndrome), cachexia (e.g. cachexia due to infection, carcinomatous cachexia, cachexia due to aquired immunodificiency syndrome), cancer, Addison's disease, Creutzfeldt-Jakob disease, virus infection (e.g. infection of virus such as cytomegalovirus, influenzavirus, herpes etc.), disseminated intravascular coagulation. The antiinflammatory agent of the present invention is effective in preventing or treating various inflammatory diseases, inter allia, inflammatory diseases [TNF-α inhibitor low sensitive inflammatory disease] which is not completely cured by a TNF-α inhibitor (e.g. TNF-α neutralizing antibody etc.) and inflammatory disease (fibrinogen lowering agent low sensitive inflammatory disease) which is not completely cured by a fibrinogen lowering agent.

In addition, the compound of the present invention can be also used as an agent for preventing or treating pain. Examples of pain diseases due to acute pain due to inflammation, pain associated with chronic inflammation, pain associated with acute inflammation, pain after operation (pain of incisional, deep pain, organ pain, chronic pain after operation etc.), muscular pain (muscular pain associated with chronic pain disease, shoulder stiffness etc.), arthralgia, toothache, gnathicarthralgia, headache (migraine, catatonic headache, headache associated with fever, headache associated hypertension), organ pain (cardiac pain, angina pain, abdominal pain, renal pain, ureterane pain, bladder pain), pain in obstetrics area (mittelschmerz, dysmenorrheal, labor pain), neuralgia, (disc hernia, nerve root pain, neuralgia after herpes zoster, trigeminal neuralgia), carcinomatous pain, reflex sympathetic atrophy, and complex local pain syndrome. The analgesic agent of the present invention is effective in alleviate directly and rapidly various pains such as nervous pain, carcinomatous pain and inflammatory pain, and exhibits the particularly excellent analgesic effect to patients and pathologies in which a pain sense threshold is lowered.

The analgesic agent and the antiinflammatory agent of the present invention is particularly useful as an analgesic agent for pain associated with chronic inflammation or pain associated with hyper tension, or as an agent for preventing or treating (1) arteriosclerosis including atherosclerosis, (2) vascular hypertrophy, occlusion or organ disorder after intervention, (3) reocclusion, restenosis or endothelial functional disorder after bypass operation, (4) intermittent claudication, (5) occlusive peripheral circulatory disorder, (6) inflammatory disease due to occlusive arteriosclerosis.

The analgesic agent and the antiinflammatory agent of the present invention may be used alone for therapy or may be used with other medicinal ingredients including other lipid lowering drug or cholesterol lowering drug, HMG-Co A reductase (3-hydroxy-3-methylglutaryl coenzyme A reductase) inhibitor, cardiac muscular protecting drug, coronary disease treating drug, other hypertension treating drug, chronic cardiac failure treating drug, diabetic treating drug, other insulin sensitivity improving drug, thyroid gland function lowering treating drug, nephrosis syndrome treating drug, antiinflammatory drug (NSAIDS etc.), bone disease treating drug (osteoporosis treating drug etc.) or chronic renal failure treating drug and, in this case, these compounds are preferably administered as an oral preparation, or, if necessary, may be administered in the form of a suppository as a rectal preparation. Examples of ingredients which can be combined in this case include fibrates [e.g. clofibrate, benzafibrate, gemfibrozil etc.], nicotinic acid, a derivative or an analog thereof [e.g. acipimox and probucol], bile acid binding resin [e.g. cholestyramin, colestipol etc.], a compound which inhibits cholesterol absorption [e.g. sitosterol and neomycin], squaleneepoxydase inhibitor [e.g. NB-598 and analogous compound].

Still other ingredients which can be combined are exemplified below:
hypertension treating drug: diuretic [e.g. furosemide (lasix), bumetanide (lunetoron), azosemide (Diart)], depressor [e.g. ACE inhibitor, (enalapril maleate (renivace) etc.) and Ca antagonist (manidipine hydrochloride, amlodipine besilate etc.), α or β receptor blocker etc.]
chronic cardiac failure treating drug: cardiac [e.g. cardiotonic glycoside (digoxin etc.), β receptor stimulator (catecholamine preparation such as denopamine and dobutamine) and PDE inhibitor etc.], diuretic [e.g. furosemide (lasix), spolonolactone (aldactone) etc.], ACE inhibitor, [e.g. enalapril maleate (renivace) etc.], Ca antagonist [e.g. amlodipine besilate etc.] and β receptor blocker
anti-arrhythmia drug: disopyramide, lidocaine, quinidine sulfate, flecaimide acetate, mexiletine hydrochloride, amiodarone hydrochloride, and β blocker, Ca antagonist
diabetic treating drug: actos, rosiglitazone, kinedak, penfill, humulin, euglucon, glimicron, daonil, novolin, monotard, insulins, glucobay, dimelin, rastinon, pamilcon, deamelin S, iszilin;
thyroid gland hypofunction treating drug: dry thyroid (thyreoid), levothroroxy sodium (thyradin S), Liothyronidine Sodium (triiodothyronine, tyrosine);
nephrosis syndrome treating drug: usually, for steroid therapy which is adopted as first selection, prednisolone (predonine), predonisolone sodium succinate (predonine), methylpredonisolone sodium succinate (solu-medrol), betamethasone (rinderon) and the like are used. And, for anti-coagulation therapy, anti-platelet drugs and anticoagulant drugs such as dipyridamole (persantin), dilazep hydrochloride (comelian), ticlopidine, clopidogrel, FXa inhibitor and the like are used;
HMG-Co A reductase inhibitor: cerivastatin sodium, atrovastatin, pravastatin, simvastatin, itavastatin, lovastatin, fluvastatin, (+)-3R,5S-7-[4-(4-fluorophenyl)-6-isopropyl-2-(N-methyl-N-methanesulfonylamino)pyrimidin-5-yl]-3,5-dihydroxy-6)-heptenoic acid and the like;
bone disease treating drug: calcium preparation (e.g. calcium carbonate etc.), calcitonin preparation, active vitamin D₃ preparation (e.g. alfacalcidol (alfarol etc.), calcitriol (rocaltrol) etc.), sex hormones (e.g. estrogen, estradiol etc.), hormone preparation [e.g. binding type estrogen (premarin) etc.], ipriflavone preparation (osten etc.), vitamin K₂, vitamin K₂ preparation [e.g. menatetrenone (glakay) etc.], bisphosphonic acid preparation (etidronate etc.), prostaglandin A1, fluorine compound (e.g. sodium fluoride etc.), osteogenic protein (BMP), fibroblast growth factor (FGF), platelet-derived growth factor (PDGF), transforming growth factor (TGF-β), insulin-like growth factor-1 and 2 (IGF-1, -2), parathyroid hormone (PTH), compounds described in European Application Publication EP-A1-376197 gazette, EP-A1-460488 gazette and EP-A1-719782 gazette (e.g. (2R. 4S)-(-)-N-[4-(dietoxyphosphorylmethyl)phenyl]-1,2,4,5-tetrahydro-4-methyl-7,8-methylenedioxy-5-oxo-3-benzothiepin-2-carboxamide etc.) etc.;
chronic renal failure treating drug: diuretic [e.g. furosemide (lasix), bumetanide (lunetoron), azosemide (Diart)], epressor (e.g. ACE inhibitor enalapril maleate (renivace)) and Ca antagonist (manidipine hydrochloride), α receptor blocker, when administered by combining these compounds, the drugs can be used preferably orally);
thrombus formation preventing and treating drug: blood coagulation inhibitor (e.g. heparin sodium, heparin calcium, warfarin calcium (warfarin), blood coagulation factor Xa inhibitor and drug having the function of correcting balance of coagulation fibrinolysis), thrombolytic drug [e.g. tPA, urokinase], anti-platelet drug [e.g. aspirin, sulphinepyrazolo (Anturan), dipyridamole (persantin), ticlopidine (panaldine), cilostazol(pletaal), GPIIb/IIIa antagonist (Reo Pro)]etc.
coronary vasodilator: nifedipine, diltiazem, nicorandil, nitrous acid drug etc.
cardiac muscular protecting drug: cardiac ATP-K opener, Na-H exchange inhibitor, endotherin antagonist, urotensin antagonist etc.
antiinflammatory drug : aspirin, acetoaminophen, non-steroidal antiinflammatory drug (e.g. indometacin etc.), steroid drug [e.g. dexamethasone etc.] etc.
anti-allergy drug: anti-histamine drug [e.g. clorpheniramine maleate etc.], stimulation therapeutic [e.g. bucillamine, etc.], other azelastine hydrochloride, seratrodast, tranilast, oxatomide, stronger neo-minophagen c, tranexamic acid, ketothiphen fumarate etc.
anti-tumor drug: alkylating drug, metabolism antagonist, anti-tumor antibiotic preparation, anti-tumor vegetable ingredient preparation and other anti-tumor drugs
central nerve-acting drug: antianxiety drug, hypnotic and sedative drug, anesthetic, antispasmodic, autonomic drug, anti-Parkinson drug and other pychonervous drug,
anti-rheumatism drug: disease-modifying anti-rheumatism drug, immunoregulating drug, anti-cytokine drug, steroid drug etc.
other anti-obesity drug etc.

The compound of the present invention can be used with these various drugs at the same time or at intervals.

When used by combining with these drugs, various drugs are separately or simultaneously formulated into a preparation by mixing with a pharmacologically acceptable carrier, excipient, or diluent, and can be used orally or parenterally as a pharmaceutical composition. When drugs are formulated into a preparation separately, separately formulated preparation can be administered by mixing them using a diluent upon use. However, separately formulated individual preparations may be administered to the same subject at the same time or at intervals separately. A kit product for administering separately formulated preparations by mixing them using a diluent upon use (e.g. an injection kit containing an ampoule containing individual powdery drugs and a diluent for mixing and dissolving two or more drugs upon use), and a kit product for administering separately formulated individual preparations to the same subject at the same time or intervals separately (e.g. a tablet kit for administering two or more kinds of tablets at the same time or at intervals separately, in which tablets containing individual drugs are placed in the same or separate bag(s) and, if necessary, a column describing times for administering drugs is made) are included in the drug of the present invention.

The present invention will be explained specifically below by way of Examples and Experimental Examples, but the present invention is not limited by them.

### Best Mode for Carrying Out the Invention

### Example

An analgesic agent and an antiinflammatory agent comprising a compound having the All antagonistic activity in the present invention or a salt thereof as an active ingredient can be prepared, for example, by the following formulation.

### Example 1

0.25g of 2-Ethoxy-1-[[2'-(5-oxo-2,5-dihydro-1,2,4-oxadiazol-3-yl)biphenyl-4-yl]methyl]-1H-benzimidazole-7-carboxylic acid (hereinafter, abbreviated as compound A) and 2.25g of a lactic acid-glycolic acid copolymer (manufactured by Wako Pure Chemical Industries, Ltd., lactic acid/glycolic acid=75/25 (% by mol), weight average molecular weight 10,700, number average molecular weight 6,100, number average molecular weight by terminal group quantitation 3,770) were dissolved in a mixed solution of 3.5ml of dichloromethane and 1.5ml of methanol, pored into 500ml of a 0.1% (w/w) aqueous polyvinyl alcohol solution which had been pre-regulated at 18°C, and formulated into an O/W emulsion at 7,000rpm using a turbine-type homomixer. This O/W emulsion was stirred at room temperature for 3 hours to evaporate the dichloromethane and the methanol, and an oily phase was solidified, which was trapped at 2,000rpm using a centrifuge. This was dispersed into distilled water again, and centrifuged, and the released drug and the like were washed. The trapped microcapsule was dispersed again by adding a small amount of distilled water, and lyophilized to obtain a powder. A recovery rate was 69%, an encapsulation rate of the compound A into the microcapsule was 92%, and the content of the compound A in the microcapsule was 9.2%.

### Example 2

A solution obtained by dissolving 0.25g of a disodium salt of the compound A in 0.4ml of distilled water, and a solution obtained by dissolving 2.25g of a lactic acid-glycolic acid copolymer (same as in Example 1) in 4ml of dichloromethane were mixed, and emulsified with a homogenizer to form a W/O emulsion. Then, this W/O emulsion was pored into 500ml of a 0.1% (w/w) aqueous polyvinyl alcohol solution which had been pre-regulated at 18°C, and formulated into a W/O/W emulsion at 7,000rpm using a turbine-type homomixer. This W/O/W emulsion was stirred at room temperature for 3 hours to evaporate the dichloromethane, an oily phase was solidified, and trapped at 2,000rpm using a centrifuge. This was dispersed into distilled water again, and centrifuged, and a released drug and the like were washed. The trapped microcapsule was dispersed again by adding a small amount of distilled water, and lyophilized to obtain a powder. A recovery rate was 50%, an encapsulation rate of the compound A into the microcapsule was 37%, and the content of the compound A in the microcapsule was 3.7%.

### Example 3

0.4g of the compound A and 1.6g of a lactic acid polymer ethyl ester (manufactured by Wako Pure Chemical Industries, Ltd., a biodegradable polymer in which a terminal carboxy group of a lactic acid polymer is ethylesterified, weight average molecular weight 10,200, number average molecular weight 5,680) were dissolved in a mixed solution of 3.5ml of dichloromethane and 2.5ml of methanol, the solution was pored into 800ml of a 5% mannitol-containing 0.1% (w/w) aqueous polyvinyl alcohol solution which had been pre-regulated at 18°C, and formulated into an O/W emulsion at 7,000rpm using a turbine-type homomixer. This O/W emulsion was stirred at room temperature for 3 hours to evaporate the dichloromethane and the methanol, an oily phase was solidified, and trapped at 2,000rpm using a centrifuge. This was dispersed into distilled water again, and further centrifuged, a released drug and the like were washed. The trapped microcapsule was dispersed again by adding a small amount of distilled water, and lyophilized to obtain a powder. A recovery rate was 83%, an encapsulation rate of the compound A into the microcapsule was 86%, and the content of the compound A in the microcapsule was 17.1%.

### Example 4

0.6g of 2-Ethoxy-1-[[2'-(IH-tetrazol-5-yl)biphenyl-4-yl]methyl]benzimidazole-7-carboxylic acid (hereinafter, abbreviated as compound B) and 0.09g of zinc oxide having a particle diameter of 0.02µm were added to a solution obtained by dissolving 2.4g of a lactic acid-glycolic acid copolymer (manufactured by Wako Pure Chemical Industries, Ltd., lactic acid/glycolic acid 75/25 (% by mol), weight average molecular weight 14,000, number average molecular weight 4,200, number average molecular weight by terminal group quantitation 4,090) in 4.5ml of dichloromethane and 1ml of ethanol, and the mixture was shaken and stirred at room temperature for 12 hours to obtain a slightly whitened solution. This solution was pored into 400ml of a 0.1% by weight aqueous polyvinyl alcohol solution which had been pre-regulated at 15°C, and formulated into an O/W emulsion at 7,000rpm using a turbine-type homomixer. This O/W emulsion was stirred at room temperature for 3 hours to evaporate the dichloromethane and the ethanol, an oily phase was solidified, and trapped at 2,000rpm using a centrifuge. This was dispersed again in distilled water, and further centrifuged, and a released drug and the like were washed. The trapped microcapsule was dispersed again by adding distilled water in which a small amount of mannitol was dissolved, and lyophilized to obtain a powder. An encapsulation rate of the compound B into the microcapsule was 97%, and the content of the compound B in the microcapsule was 18.8%.

### Example 5

According to the same manner as that of Example 1 except that an amount of zinc oxide was changed to 0.057g, a microcapsule was obtained. An encapsulation rate or the compound B into the microcapsule was 97%, and the content of the compound B in the microcapsule was 19.0%.

### Example 6

According to the same manner as that of Example 1 except that an amount of the compound B, an amount of zinc oxide and an amount of a lactic acid-glycolic acid copolymer were changed to 0.9g, 2.1g and 0.12g, respectively, a microcapsule was obtained. An encapsulation rate of the compound B into the microcapsule was 96%, and the content of the compound B in the microcapsule was 27.8%.

### Examples 7

According to the same manner as that of Example 3 except that an amount of zinc oxide was changed to 0.18g, a microcapsule was obtained. An encapsulation rate of the compound B into the microcapsule was 92%, and the content of the compound B in the microcapsule was 26.2%.

### Example 8

1.8g of the compound B and 0.3g of zinc oxide having a particle diameter of 0.02µm were added to a solution obtained by dissolving 4-2g of a lactic acid-glycolic acid copolymer (manufactured by Wako Pure Chemical Industries, Ltd., lactic acid/glycolic acid 75/25 (% by mol) weight average molecular weight 14,000, number average molecular weight 4,200, number average molecular weight by terminal group quantitation 4,090) in 9ml of dichloromethane and 1.5ml of ethanol, and shaken and stirred at room temperature for 12 hours to obtain a slightly whitened solution. This solution was pored into 800ml of a 0.1% by weight aqueous polyvinyl alcohol solution which had been pre-regulated at 15°C, and formulated into an O/W emulsion at 7,000rpm using a turbine-type homomixer. This O/W emulsion was stirred at room temperature for 3 hours to evaporate the dichloromethane and the methanol, an oily phase was solidified, and trapped at 2,000rpm using a centrifuge. This was dispersed into distilled water again, and further centrifuged, and a released drug and the like were washed. The trapped microcapsule was dispersed again by adding distilled water in which a small amount of mannitol was dissolved, and lyophilized to obtain a powder. An encapsulation rate of the compound B into the microcapsule was 94%, and the content of the compound B in the microcapsule was 26.8%.

### Example 9

0.3g of the compound A and 0.05g of zinc oxide having a particle diameter of 0.02µm were added to a solution obtained by dissolving 0.7g of a lactic acid-glycolic acid copolymer (manufactured by Wako Pure Chemical Industries, Ltd., lactic acid/glycolic acid 75/25 (% by mol) weight average molecular weight 14,000, number average molecular weight 4,200, number average molecular weight by terminal group quantitation 4,090) in 1.5ml of dichloromethane and 1ml of methanol, and shaken and stirred at room temperature for 12 hours to obtain a slightly whitened solution. This solution was pored into 300ml of a 0.1% by weight aqueous polyvinyl alcohol solution which had been pre-regulated at 15°C, and formulated into an O/W emulsion at 6,500rpm using a turbine-type homomixer. This O/W emulsion was stirred at room temperature for 3 hours to evaporate the dichloromethane and the ethanol, an oily phase was solidified, and trapped at 2,000rpm using a centrifuge. This was dispersed into distilled water again, and further centrifuged, and a released drug and the like were washed. The trapped microcapsule was dispersed again by adding distilled water in which a small amount of mannitol was dissolved, and lyophilized to obtain a powder. An encapsulation rate of the compound B into the microcapsule was 91%, and the content of the compound B in the microcapsule was 29.5%.

### Example 10

1g of the compound B and 0.18g of zinc oxide having a particle diameter of 0.02µm were added to a solution obtained by dissolving 1.8g of a lactic acid-glycolic acid copolymer (manufactured by Wako Pure Chemical Industries, Ltd., lactic acid/glycolic acid 75/25 (% by mol) weight average molecular weight 14,000, number average molecular weight 4,200, number average molecular weight by terminal group quantitation 4,090) in 5ml of dichloromethane, and emulsion-mixed for 60 seconds with a miniature homogenizer to obtain a white cloudy dispersion. This dispersion was pored into 400ml of a 0.1% by weight aqueous polyvinyl alcohol solution which had been pre-regulated at 15°C, and formulated into an O/W emulsion at 8,000rpm using a turbine-type homomixer. This O/W emulsion was stirred at room temperature for 3 hours to evaporate the dichloromethane, an oily phase was solidified, and trapped at 2,000rpm using a centrifuge. This was dispersed into distilled water again, and further centrifuged, and a released drug and the like were washed. The trapped microcapsule was dispersed again by adding distilled water in which a small amount of mannitol was dissolved, and lyophilized to obtain a powder. An encapsulation rate of the compound B into the microcapsule was 96%, and the content of the compound B in the microcapsule was 32.0%.

### Example 11

According to the same manner as that of Example 7 except that 0.8ml of ethanol was added to dichloromethane, shaken and stirred at room temperature for 12 hours to obtain a slightly whitened solution, and this solution was used, a microcapsule was obtained. An encapsulation rate of the compound B into the microcapsule was 95%, and the content of the compound B in the microcapsule was 32.0%.

### Example 12

0.9g of 1-(Cyclohexyloxycarbonyloxy)ethyl 2-ethoxy-1-[[2'-(IH-tetrazol-5-yl)biphenyl-4-yl]methyl]benzimidazole-7-carboxylate (hereinafter, abbreviated as compound C) and 2.1 of a lactic acid-glycolic acid copolymer (manufactured by Wako Pure Chemical Industries, Ltd., lactic acid/glycolic acid 75/25 (% by mol) weight average molecular weight 14,000, number average molecular weight 4,200, number average molecular weight by terminal group quantitation 4,090) were dissolved in a mixed solvent of 4.5ml of dichloromethane and 0.7ml of ethanol. To this solution was added 0.15g of zinc oxide having a particle diameter of 0.02 µm, and shaken and stirred at room temperature for 12 hours to obtain a slightly whitened solution. This solution was pored into 400ml of a 0.1% by weight aqueous polyvinyl alcohol solution which had been pre-regulated at 15°C, and formulated into an O/W emulsion at 7,500rpm using a turbine-type homomixer. This O/W emulsion was stirred at room temperature for 3 hours to evaporate the dichloromethane and the ethanol, an oily phase was solidified, and trapped at 2,000rpm using a centrifuge. This was dispersed into distilled water again, and further centrifuged, and a released drug and the like were washed. The trapped microcapsule was dispersed again by adding distilled water in which a small amount of mannitol was dissolved, and lyophilized to obtain a powder. An encapsulation rate of the compound C into the microcapsule was 96%, and the content of the compound C in the microcapsule was 27.4 %.

### Example 13

According to the same manner as that of Example 12 except that zinc oxide was not added, a microcapsule was prepared. An encapsulation rate of the compound C into the microcapsule was 98%, and the content of the compound C in the microcapsule 30.0%.

### Example 14

1.2g of the compound C and 1.8g of a lactic acid-glycolic acid copolymer (manufactured by Wako Pure Chemical Industries, Ltd., lactic acid/glycolic acid 75/25 (% by mol) weight average molecular weight 14,000, number average molecular weight 4,200, number average molecular weight by terminal group quantitation 4,090) were dissolved in 5ml of dichloromethane. To this solution was added 0.18g of zinc oxide having a particle diameter of 0.02µm, and shaken and stirred at room temperature for 1 hour to obtain a slightly whitened solution. This solution was pored into 400ml of a 0.1% by weight aqueous polyvinyl alcohol solution which had been pre-regulated at 15°C, and formulated into an O/W emulsion at 8,000rpm using a turbine-type homomixer. This O/W emulsion was stirred at room temperature for 3 hours to evaporate the dichloromethane, an oily phase was solidified, and trapped at 2,000rpm using a centrifuge. This was dispersed into distilled water again, and further centrifuged, and a released drug and the like were washed. The trapped microcapsule was dispersed again by adding distilled water in which a small amount of mannitol was dissolved, and lyophilized to obtain a powder. An encapsulation rate of the compound C into the microcapsule was 95%, and the content of the compound C in the microcapsule was 35.9 %.

### Example 15

According to the same manner as that of Example 4 except that zinc oxide was not added, a microcapsule was prepared. An encapsulation rate of the compound B into the microcapsule was 99%, and the content of the compound B in the microcapsule 19.8%

### Example 16

According to the same manner as that of Example 9 except that zinc oxide was not added, a microcapsule was prepared. An encapsulation rate of the compound A into the microcapsule was 95%, and the content of the compound A in the microcapsule 28.4%.

### Example 17

2g of 2-Ethoxy-1-[[2'-(1H-tetrazol-5-yl)biphenyl-4-yl]methyl]benzimidazole-7-carboxylic acid (compound B) and 0.36g of zinc oxide (manufactured by Wako Pure Chemical Industries, Ltd., TYPE V) were added to a solution obtained by dissolving 3.6g of a lactic acid-glycolic acid copolymer (manufactured by Wako Pure Chemical Industries, Ltd., lactic acid/glycolic acid 75/25 (% by mol) weight average molecular weight 14,000, number average molecular weight 4,200, number average molecular weight by terminal group quantitation 4,090) in 11ml of dichloromethane and 0.4 ml of ethanol, and shaken and stirred at room temperature for 14 hours to obtain a whitened solution. This solution was pored into 800ml of a 0.1% by weight aqueous polyvinyl alcohol solution which had been pre-regulated at 15°C, and formulated into an O/W emulsion at 8,500rpm using a turbine-type homomixer. This O/W emulsion was stirred at room temperature for 3 hours to evaporate the dichloromethane and the ethanol, an oily phase was solidified, and trapper at 2,000rpm using a centrifuge. This was dispersed into distilled water again, and further centrifuged, and a released drug and the like were washed. The trapped microcapsule was dispersed again by adding distilled water in which a small amount of mannitol was dissolved, and lyophilized to obtain a powder. An encapsulation rate of the compound B into the microcapsule was 98%, and the content of the compound B in the microcapsule was 33.0%.

### Example 18

According to the same manner as that of Example 17 except that 0.4ml of distilled water was added, and shaking and stirring for 14 hours was changed to dispersing (emulsification) mixing with a solid (compound B and zinc oxide) at the same rotation number for 1 minute using a homogenizer, a microcapsule was obtained. An encapsulation rate of the compound B into the microcapsule was 97%, and the content of the compound B in the microcapsule was 32.6%.

### Example 19

According to the same manner as that of Example 18 except that an amount of distilled water to be added was changed to 0.08ml, a microcapsule was obtained. An encapsulation of the compound B into the microcapsule was 97%, and the content of the compound B in the microcapsule was 32.5%.

### Example 20

4g of the compound B and 0.72g of zinc oxide (manufactured by Wako Pure Chemical Industries, Ltd., TYPE V) were added to a solution obtained by dissolving 7.2g of a lactic acid-glycolic acid copolymer (lactic acid/glycolic acid 75/25 (% by mol), weight average molecular weight 10,600) in 22ml of dichloromethane and 0.8ml of ethanol, 0.16ml of distilled water was added thereto and, immediately, the materials were dispersion (emulsification)-mixed with a homogenizer under the same conditions as those of Example 18 to obtain a whitened solution. This was spread on a planar plate at a form of circle having a radius of about 5cm, and this was dried under reduced pressure at room temperature for 15 hours to obtain a dried material. This dried material was roughly ground and classified on a sieve having a pore diameter of 250µm, 5g of the resulting dried material was mixed with 0.4g of mannitol, and air-ground at an air pressure of 2kg/cm² using a jet mill apparatus (manufactured by Seishinkigyo, A-OJET), to obtain a fine particle having an average particle diameter of 21µm. The content of the compound B in the fine particle was 31.0%.

### Example 21

The whitened solution obtained by dispersing (emulsification) mixing under the same formulation and procedures as those of Example 20 was spray dried (manufactured by Niro Japan, Mobile Miner) under the following conditions, to obtain a fine particle having an average particle diameter of 32µm as a dried material under cyclone.

Spraying format: two-fluid nozzle (nozzle diameter 1.2mm)
Air pressure: 1kg/cm²
Drying chamber inlet temperature: 90°C
Drying chamber outlet temperature: 40 to 43°C
The content of the compound B in the resulting fine particle was 28.1%.

### Example 22, Capsule

| | |
|---|---|
| (1) Candesartan cilexetil | 30mg |
| (2) Lactose | 90mg |
| (3) Microcrystalline cellulose | 70mg |
| (4) Magnesium stearate | 10mg |
| One capsule | 200mg |

(1), (2), (3) and 1/2 of (4) are kneaded, and granulated. To this is added the remaining (4), and the whole is encapsulated into a gelatin capsule.

### Example 23, Tablet

| | |
|---|---|
| (1) Candesartan cilexetil | 30mg |
| (2) Lactose | 35mg |
| (3) Corn starch | 150mg |
| (4) Microcrystalline cellulose | 30mg |
| (5) Magnesium stearate | 5mg |
| One tablet | 250mg |

(1),(2),(3), 2/3 of (4) and 1/2 of (5) are kneaded, and granulated. The remaining (4) and (5) are added to this granule, and press-molded into a tablet.

### Example 24, Capsule

| | |
|---|---|
| (1) 2-Ethoxy-1-[[2'-(5-oxo-2,5-dihydro-1,2,4-oxadiazol-3-yl)biphenyl-4-yl]methyl]-1H-benzimidazole-7- | |
| carboxylic acid | 30mg |
| (2) Lactose | 90mg |
| (3) Microcrystalline cellulose | 70mg |
| (4) Magnesium stearate | 10mg |
| One capsule | 200mg |

(1), (2), (3) and 1/2 of (4) are kneaded, and granulated. To this is added the remaining (4), and the whole is encapsulated into a gelatin capsule.

### Example 25, Tablet

| | |
|---|---|
| (1) 2-Ethoxy-1-[[2'-(5-oxo-2,5-dihydro-1,2,4- | |
| oxadiazol-3-yl)biphenyl-4-yl]methyl]-1H-benzimidazole-7- | |
| carboxylic acid | 30mg |
| (2) Lactose | 35mg |
| (3) Corn starch | 150mg |
| (4) Microcrystalline cellulose | 30mg |
| (5) Magnesium stearate | 5mg |
| One tablet | 250mg |

(1), (2), (3), 2/3 of (4) and 1/2 of (5) are kneaded, and granulated. The remaining (4) and (5) are added to this granule, and press-molded into a tablet.

### Experimental Example 1, Antiinflammatory action

### Carrageenin edema method

Method: SD strain male rats (6 weeks old, Nihon Clea) were used at 6 animals per group. The carrageenin edema method was performed according to a method of Winter et al. (Proc. Soc. exp. Biol. Med., 111, 544-547, 1962). After a volume of a right rear limb plantar part was measured, a specimen [compound A: 2-ethoxy-1-[[2'-(5-oxo-2,5-dihydro-1,2,4-oxadiazol-3-yl) biphenyl-4-yl]methyl]-1H-benzimidazole-7-carboxylic acid] was orally administered (1ml/100g and water was additionally added to a total amount of 5ml/rat. After one hour, 0.05ml of a 1% carrageenin physiological saline solution was subcutaneously injected to induce edema. Whereby, a volume of a right rear limb plantar part was measured after 3 hours. The effect of a specimen was obtained by a difference of a limb volume between before carrageenin injection and after 3 hours, and calculating an inhibition rate (%) relative to a control group. The results are shown in Table 1.

**[Table 1]**

| | | |
|---|---|---|
| Compound | Dose (mg/kg) | Inhibition rate (%) |
| Compound A | 30 | 34* |

| | | |
|---|---|---|
| *: p<0.05 vs control (Dunnett's test) | | |

It is seen from the results of Table 1 that the compound of the present invention has the excellent antiinflammatory activity (carrageenin edema inhibiting action).

### Experimental Example 2, Analgesic action

### Acetic acid writhing method

Method: ICR strain male mice (5 weeks old, Nihon Clea) were used at 10 animals per group, and a specimen [compound A: 2-ethocy-1-[[2'-(5-oxo-2,5-dihydro-1,2,4-oxadiazol-3-yl)biphenyl-4-yl]methyl]-1H-benzimidazole-7-carboxylic acid] was orally administered (0.2ml/10g). After 30 minutes, a 0.6% acetic acid solution was intraperitoneally injected (0.1ml/10g) and, immediately, mice were placed into a transparent acryl resin observation cage, a time of writhing and stretching induced for 20 minutes therefrom was counted, and an inhibition rate (%) was calculated from average times of a control group and a specimen administration group. The results are shown in Table 2.

### Results:

**[Table 2]**

| | | |
|---|---|---|
| Compound | Dose (mg/kg) | Inhibition rate (%) |
| Compound A | 30 | 52* |

| | | |
|---|---|---|
| *: p<0.05 vs control (Dunnett's test) | | |

It is seen from the results of Table 2 that the compound of the present invention has the excellent analgesic action (acetic acid writhing inhibiting action).

### Experimental Example 3, Pain threshold improving action in SHRSP rat (easily cerebral apoplexy developing spontaneous hypertension rat)

SHRSP strain male rats (27 weeks old, Nihon Clea, 6 animals per group) were used. A right rear limb plantar part was pressed using a balance-type pressing apparatus, and a measured value when a false escape reaction was exhibited was used as a pain threshold value. Measurement of a pain threshold value was performed according to a Randall & Selitto's method (Arch. Int. Pharmacodyn. Ther. 111, 409-419, 1957). Immediately after therefrom, a compound A [2-ethoxy-1-[[2'-(5-oxo-2,5-dihydro-1,2,4-oxadoazol-3-yl)biphenyl-4-yl]methyl]-1H-benzimidazole-7-carboxylic acid] was orally administered (0.5ml/100g, b.w.) and, one hour after, a pain threshold value was measured. Only a solvent was orally administered to a control group. Regarding the effect of a specimen, drug efficacy assessment was performed by threshold pressure of a control group and a specimen administration group. The results are shown in Table 3.

**[Table 3]**

| Action of compound A on pain threshold value at SHRSP rat right rear limb plantar part | | |
|---|---|---|
| Compound Pain threshold (g) | | |
| A (mg/kg) | Before administration of compound A | 1 hour after administration of compound A |
| Solvent control | 212 | 205 |
| 1 | 210 | 277* |
| 3 | 213 | 352* |
| 10 | 212 | 430* |
| 30 | 214 | 470* |

| | | |
|---|---|---|
| *: p ≤ 0.025 vs control (Williams test) | | |

It is seen from the results of Table 3 that the compound of the present invention has the excellent analgesic action.

### Industrial Applicability

The agent of the present invention exhibits the excellent analgesic and antiinflammatory actions and is useful as an analgesic agent and an antiinflammatory agent.

## Claims

1. An analgesic agent comprising 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

2. An antiinflammatory agent comprising 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

3. An analgesic agent comprising a compound having the angiotensin II antagonistic activity selected from Losartan, Candesartan, Olmesartan and Tasosartan, a prodrug thereof or a salt thereof.

4. An antiinflammatory agent comprising a compound having the angiotensin II antagonistic activity selected from Candesartan, Telmisartan, Olmesartan and Tasosartan, a prodrug thereof or a salt thereof.

5. An analgesic agent for pain associated with chronic inflammation or headache associated with hypertension, comprising a compound having the angiotensin II antagonistic activity, a prodrug thereof a salt thereof.

6. An agent for improving a pain sense threshold comprising a compound having the angiotensin II antagonistic activity, a prodrug thereof or a salt thereof.

7. An agent for preventing or treating (1) arteriosclerosis including atherosclerosis, (2) vascular hypertrophy, occlusion or organ disorder after intervention, (3) reocclusion, restenosis or endothelial functional disorder after bypass operation, (4) intermittent claudication, (5) occlusive peripheral circulation disorder, or (6) inflammatory disease or pain due to occlusive arteriosclerosis, comprising a compound having the angiotensin II antagonistic activity, a prodrug thereof or a salt thereof.

8. The agent according to any one of claims 5 to 7, wherein the compound having the angiotensin II antagonistic activity is a non-peptidic compound.

9. The agent according to any one of claims 5 to 7, wherein the compound having the angiotensin II antagonistic activity is a compound having an oxygen atom in the molecule.

10. The agent according to any one of claims 5 to 7, wherein the compound having the angiotensin II antagonistic activity is a compound having an ether linkage or a carbonyl group.

11. The agent according to any one of claims 5 to 7, wherein the compound having the angiotensin II antagonistic activity is a compound represented by the formula: 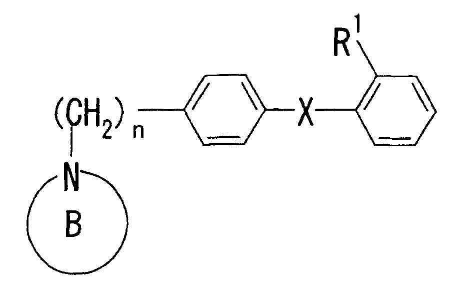 wherein a ring B denotes an optionally substituted nitrogen-containing heterocycle, R¹ denotes a group which can forms an anion or a group which can be converted into an anion, X denotes that the phenylene group and the phenyl group are bound directly or through a spacer having a chain length of 1 or 2 atoms, and n denotes an integer of 1 or 2.

12. The agent according to claim 11, wherein the ring B is an optionally substituted nitrogen-containing aromatic heterocycle.

13. The agent according to claim 11, wherein the ring B is an optionally substituted 5 or 6-membered nitrogen-containing heterocycle.

14. The agent according to claim 11, wherein the ring B is an optionally substituted imidazole ring.

15. The agent according to any one of claims 5 to 7, wherein the compound having the angiotensin II antagonistic activity is a compound represented by the formula (I): 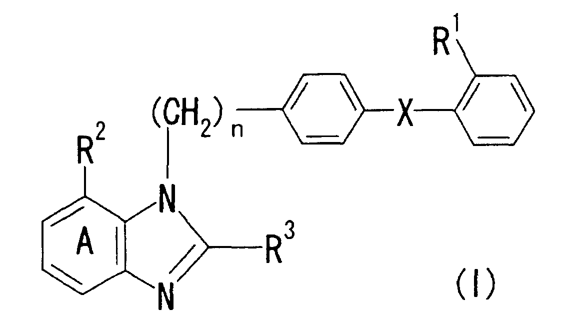 wherein R¹ denotes a group which can form an anion or a group which can be converted into an anion, X denotes that the phenylene group and the phenyl group are bound directly or through a spacer having a chain length of 1 or 2 atoms, n denotes 1 or 2, a ring A denotes a benzene ring optionally further having a substituent, R² denotes a group which can form an anion or a group which can be converted into an anion, and R³ denotes a hydrocarbon residue which may be bound via a hetero atom and which may have a substituent.

16. The agent according to any one of claims 5 to 7, wherein the compound having the angiotensin II antagonistic activity is Losartan, Eprosartan, Candesartan Cilexetil, Candesartan, Valsartan, Telmisartan, Irbesartan, Olmesartan or Tasosartan.

17. The agent according to any one of claims 5 to 7, wherein the compound having the angiotensin II antagonistic activity is 2-ethoxy-1-[[2'-(1H-tetrazol-5-yl)biphenyl-4-yl]methyl]benzimidazole-7-carboxylic acid.

18. The agent according to any one of claims 5 to 7, wherein the compound having the angiotensin II antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate.

19. The agent according to any one of claims 5 to 7, wherein the compound having the angiotensin II antagonistic activity is 2-ethoxy-1-[[2'-(5-oxo-2,5-dihydro-1,2,4-oxadiazol-3-yl)biphenyl-4-yl]methyl]-1H-benzimidazole-7-carboxylic acid.

20. A method for preventing or treating pain, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

21. A method for preventing or treating inflammatory disease, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

22. A method for preventing or treating pain, which comprises administering an effective amount of a compound having the angiotensin II antagonistic activity selected from Losartan, Candesartan, Olmesartan and Tasosartan, a prodrug thereof or a salt thereof to a mammal.

23. A method for preventing or treating inflammatory disease, which comprises administering an effective amount of a compound having the angiotensin II antagonistic activity selected from Candesartan, Telmisartan, Olmesartan and Tasosartan, a prodrug thereof or a salt thereof to a mammal.

24. A method for preventing or treating pain associated with chronic inflammation or headache associated with hypertension, which comprises administering an effective amount of a compound having the angiotensin II antagonistic activity, a prodrug thereof or a salt thereof to a mammal.

25. A method for improving a pain sense threshold, which comprises administering an effective amount of a compound having the angiotensin II antagonistic activity, a prodrug thereof or a salt thereof to a mammal.

26. A method for preventing or treating (1) arteriosclerosis including atherosclerosis, (2) vascular hypertrophy, occlusion or organ disorder after intervention, (3) reocclusion, restenosis or endothelial functional disorder after bypass operation, (4) intermittent claudication, (5) occlusive peripheral circulation disorder, or (6) inflammatory disease or pain due to occlusive arteriosclerosis, which comprises administering a compound having the angiotensin II antagonistic activity, a prodrug thereof or a salt thereof to a mammal.

27. An use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for preparation of an analgesic agent.

28. An use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for preparation of an antiinflammatory agent.

29. An use of a compound having the angiotensin II antagonistic activity selected from Losartan, Candesartan, Olmesartan and Tasosartan, a prodrug thereof or a salt thereof for preparation of analgesic agent.

30. An use of a compound having the angiotensin II antagonistic activity selected from Candesartan, Telmisartan, Olmesartan and Tasosartan, a prodrug thereof or a salt thereof for preparation of an antiinflammatory agent.

31. An use of a compound having the angiotensin II antagonistic activity, a prodrug thereof or a salt thereof for preparation of an analgesic agent for pain associated with chronic inflammation or headache associated with hypertension.

32. An use of a compound having the angiotensin II antagonistic activity, a prodrug thereof or a salt thereof for preparation of an agent for improving pain sense threshold.

33. An use of a compound having the angiotensin II antagonistic activity, a prodrug thereof or a salt thereof for preparation of an agent for preventing or treating (1) arteriosclerosis including atherosclerosis, (2) vascular hypertrophy, occlusion or organ disorder after intervention, (3) reocclusion, restenosis or endothelial functional disorder after bypass operation, (4) intermittent claudication, (5) occlusive peripheral circulation disorder, or (6) inflammatory disease or pain due to occlusive arteriosclerosis.
